Europäisches Patentamt

European Patent Office

Office européen des brevets

(11) Publication number: **0 062 971**
**B1**

(12) # EUROPEAN PATENT SPECIFICATION

(45) Date of publication of patent specification: **07.03.90**

(21) Application number: **82301309.9**

(22) Date of filing: **15.03.82**

(51) Int. Cl.⁵: **C 12 N 15/00,** C 12 R 1/01 //
C12R1/26, C12R1/05,
C12R1/38

(54) **Genetically modified microorganisms.**

(30) Priority: **27.03.81 GB 8109678**
**30.03.81 GB 8109919**
**22.04.81 GB 8112446**
**07.09.81 GB 8126979**

(43) Date of publication of application:
**20.10.82 Bulletin 82/42**

(45) Publication of the grant of the patent:
**07.03.90 Bulletin 90/10**

(84) Designated Contracting States:
**AT BE CH DE FR GB IT LI LU NL SE**

(56) References cited:
EP-A-0 001 931     EP-A-0 060 057
EP-A-0 018 218     FR-A-2 442 054
EP-A-0 028 033     GB-A-2 003 926
EP-A-0 032 134

NATURE, vol. 287, no. 5781, 1980, Chesham,
Bucks. (GB); G.ALLEN et al.: "A family of
structural genes for human lymphoblastoid
(leucocyte-type) interferon", pp. 408-411

NATURE, vol. 293, 1981; R.A.HITZMAN et al.:
"Expression of a human gene for interferon in
yeast", pp. 717-722

NATURE, vol. 292, 1981; M.D.EDGE et al.: "Total
synthesis of a human leucocyte interferon
gene", pp. 756-762

(73) Proprietor: **IMPERIAL CHEMICAL INDUSTRIES
PLC**
**Imperial Chemical House Millbank**
**London SW1P 3JF (GB)**

(73) Proprietor: **UNIVERSITY OF LEICESTER**
**University Road**
**Leicester, LE1 7RH (GB)**

(72) Inventor: **Atherton, Keith Thurston**
**Tarvin Close**
**Runcorn Cheshire (GB)** ·
Inventor: **De Maeyer, Edward**
**34 Avenue St. Laurent Batiment 2**
**F-91400 Orsay (FR)**
Inventor: **Edge, Michael Derek**
**18 Bowness Court**
**Congleton Cheshire (GB)**
Inventor: **Markham, Alexander Fred**
**307 Market Street**
**Droylsden Manchester (GB)**
Inventor: **Meacock, Peter Anthony**
**91 Fleckney Road**
· **Kibworth Beauchamp Leicestershire (GB)**
Inventor: **Windass, John David**
**6 Nursery Hollow**
**Glen Parva Leicestershire (GB)**

Courier Press, Leamington Spa, England.

(56) References cited:
GENE, vol. 10, 1980, pp. 1-10
GENE, vol. 11, 1980, pp. 181-186
NATURE, vol. 284, 1980, pp. 316-320
SCIENCE, vol. 209, 1980, pp. 1343-1347
NATURE, vol. 287, 1980, pp. 396-401

(74) Representative: Thomas, Ieuan et al
Imperial Chemical Industries PLC Legal
Department: Patents PO Box 6 Bessemer Road
Welwyn Garden City Herts, AL7 1HD (GB)

**Description**

This invention relates to a process for the preparation of genetic material coding for a compound having interferon activity.

The techniques of recombinant genetic technology have been discussed in the literature, including the patent literature, and usually comprise, at their simplest, the steps of obtaining, from a donor cell, the DNA coding for a desired metabolite, cleaving a suitable vector to provide an insertion site wherein the aforesaid DNA may be incorporated into the vector, inserting the aforesaid DNA into the vector to form a recombinant DNA molecule, transforming a host bacterium, to which the aforesaid DNA is foreign, with the recombinant DNA molecule and culturing the transformed bacterium to cause it to produce the desired metabolite.

Interferons are known compounds which are produced by a variety of living cells both in vivo and in vitro in tissue cultures in response to certain inducers. Interferons are believed to have anti-viral activity and consequently are potentially important in the treatment of virus induced disease but their potential has not yet been developed because they are produced only in small quantity by existing techniques. The production of interferons by genetically modified bacteria has, therefore, been suggested as a means of meeting needs for the material.

Information on the biological properties of interferons has come predominantly from studies of material produced by cell culture techniques. Recent results using recombinant DNA techniques have shown that human leucocyte interferon (IFN-α) is a family of at least 8 different molecules (Goeddel et al, Nature, 1981, 290, pages 20—26). Furthermore, Allen and Fantes (Nature, 1980, 287, pages 408—411) have demonstrated that microheterogeneities in interferon preparations from Namalwa cells can be ascribed to the presence of at least five homologous proteins with different amino acid sequences and hence to different structural genes. Despite these advances, very little is known about the distinct physiological properties of individual human IFN species.

Recently, copy DNA (cDNA) coding for compounds having interferon activity has been prepared using mRNA from human leucocyte cells. Expression of the aforementioned cDNA in E. coli to produce, for example, Hu IFN-α$_1$, has been reported (Stewart et al, Gene, 1980, 11, pages 181—186). We have now found that expression of cDNA coding for compounds having interferon activity per se or capable of being modified in vivo or in vitro to provide a compound having interferon activity can be effected in methylotrophic and autotrophic organisms, and that such cDNA can be prepared using mRNA from Namalwa cells. Methylotrophic and autotrophic organisms often do not infect man and are particularly safe for large scale biosynthesis.

The availability of cloned IFN genes has facilitated the production of structural variants of interferon by the construction of hybrid genes. Cleavage at a common restriction site (Streuli et al, Proceedings of the National Academy of Sciences, USA, 1981, 78, pages 2848—2852) within the coding sequence of two different interferon genes allows fusion of the NH$_2$-terminal sequence from one with the COOH terminal sequence of the other. However, the number and type of molecules which can be synthesised by this approach is limited.

The determination of nucleic acid sequences in DNA can now be performed with precision by routine, albeit sometimes lengthy, techniques and genes of small proteins, e.g. human somatostatin, human insulin A and B chains and rat proinsulin, have been chemically synthesised.

We have now chemically synthesised genes which express compounds having interferon activity, the sequence of Hu IFN-α$_1$, reported by Mantei et al, Gene, 1980, 10, pages 1—10 and Nagata et al, Nature, 1980, 287, pages 401—408 and Hu INF-α$_2$, reported by Streuli et al, Science, 1980, 209, pages 1343—1347. These genes may be inserted into a vector and hence into a microorganism to obtain expression of the gene with the production of compounds having interferon activity by the host cell.

According to one aspect of the present invention, therefore we provide a process for the preparation of genetic material coding for a compound having interferon activity per se or capable of being modified in vivo or in vitro to provide a compound having interferon activity characterised in that the process comprises at least the steps of

(A) chemically synthesising fragments of the genetic material each of which fragments contains four to six inclusive oligonucleotides which are alignable through hydrogen bonding to the complementary strand;

(B) preparing sequences by joining two to three appropriate fragments which are substantially free of intra- and inter-complementarity; and

(C) joining the said sequence in the appropriate order; such that the said genetic material is prepared with the proviso that sites recognisable by restriction enzymes used in the latter stages of the process are avoided.

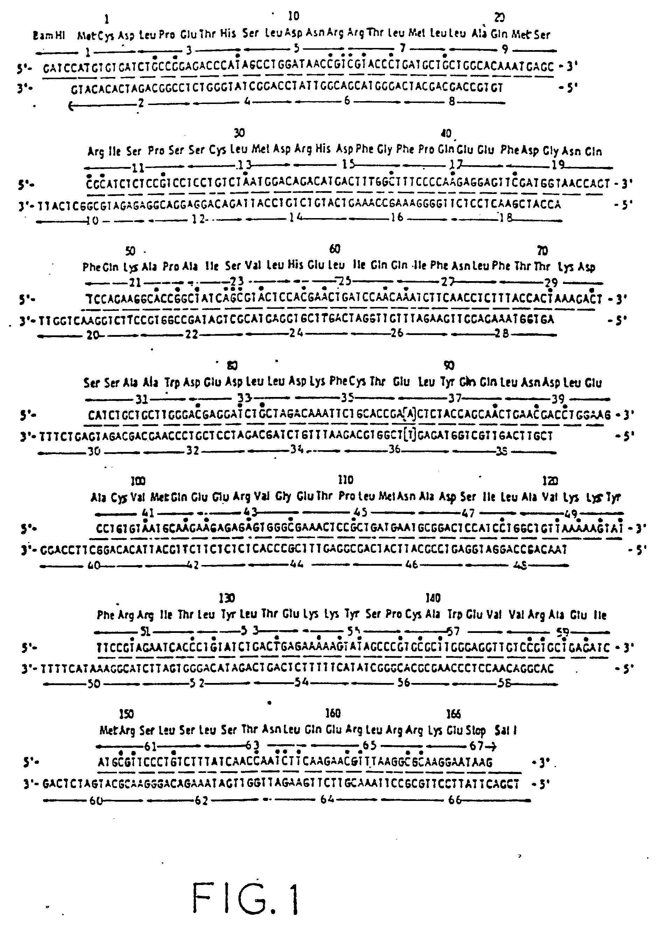

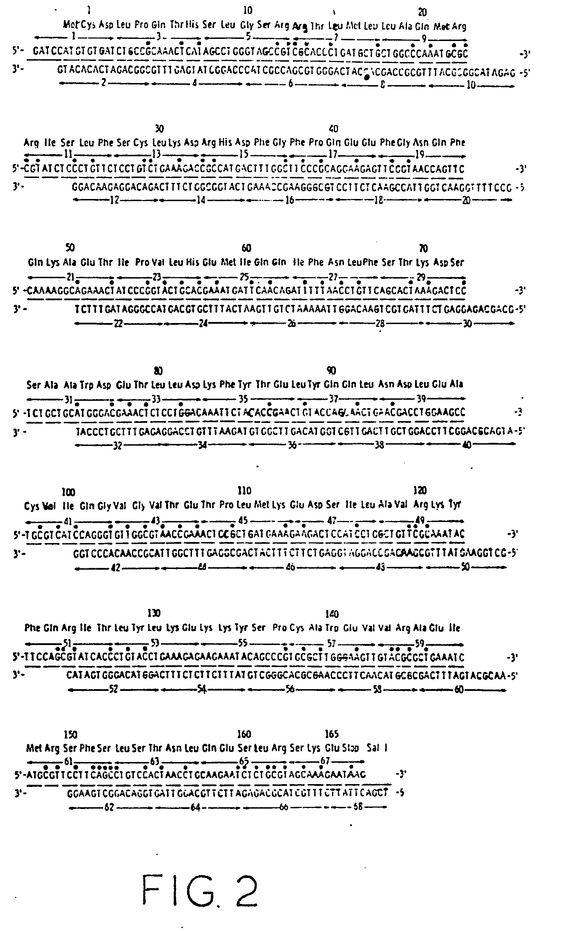

Conveniently the oligonucleotides used in step (A) of the above process are chosen from those designated 1 to 67 in Figure 1 or are chosen from those designated 1 to 68 in Figure 2; as set out hereinafter.

Conveniently the fragments used in Step (B) of the above process are chosen from those designated A to K as set out in Table 1 of Example 1, part (a) or are chosen from those designated A to M as set out in Table 7 of Example 8; as set out hereinafter.

The sequences used in Step (C) of the above process are conveniently selected from those designated I to IV in Table 2 of Example 1 or are selected from those designated I to III in Table 8 of Example 8; set out hereinafter.

We have also prepared a genetically modified microorganism capable of expressing as a metabolite a compound having interferon activity per se or capable of being modified in vivo or in vitro to provide a compound having interferon activity and comprising genetic material coding for the said metabolite, which genetic material comprises a synthetic DNA molecule or a copy DNA molecule derived from mRNA produced by transcription of a naturally occurring interferon gene, and where the said genetic material comprises a copy DNA molecule derived from mRNA produced by transcription of a naturally occurring interferon gene and the genetically modified microorganism is derived from E. coli and the naturally occurring gene is a component of Namalwa cells.

In this specification the terms and symbols listed below have the following meanings ascribed to them:—

IFN—interferon;

Hu IFN—human interferon;

DNA—deoxyribonucleic acid—genetic material of all self-replicating organisms;

cDNA—DNA formed by reverse transcription of mature messenger ribonucleic acid;

ds cDNA—double strand cDNA;

mRNA—messenger ribonucleic acid formed by transcription of the coding strand of DNA;

bp—base pair;

AS1—methylophilus methylotrophus;

$Tc^R$—tetracycline resistance;

$Ap^R$—ampicillin resistance.

By "a compound having interferon activity" we mean a polypeptide that displays a biological or immunological activity of Hu IFN. As examples of such activity we would mention inter alia antiviral, anti-proliferation (of cells) and immunomodulating activities; and the neutralisation thereof by anti-interferon sera.

In the drawings accompanying this specification:

Figure 1 illustrates the nucleotide sequence of a Hu IFN-$\alpha_1$ gene and the amino acid sequence derived from it;

Figure 2 illustrates the nucleotide sequence of a Hu IFN-$\alpha_2$ gene and the amino acid sequence derived from it;

Figure 3 is a schematic representation of the expression control region of a prokaryotic gene;

Figure 4 illustrates construction of a suitable site in a plasmid for insertion of an E. coli lac promoter therein;

Figure 5 is a diagrammatic representation of the structure of promoter vector pPM50;

Figure 6 illustrates the nucleotide sequence of a synthetic trp promoter used in the present invention;

Figure 7 is a diagrammatic representation of a promoter vector comprising a synthetic trp promoter;

Figure 8 is a diagrammatic representation of the structure of plasmids pSTP1 and pSTP2;

Figure 9 is a schematic outline of the construction of recombinant DNA molecules pIFS201—230 used in the present invention;

Figure 10 illustrates reconstruction of a Hu IFN-$\alpha_1$ gene and incorporation thereof into a vector to form pJDW22;

Figure 11 illustrates details of the construction for fusing a synthetic trp promoter and a Hu IFN-$\alpha_1$ gene to form a recombinant molecule of the present invention;

Figure 12 is a schematic representation of the preparation of oligonucleotides for use in the present invention;

Figure 13 is a schematic representation of a recombinant DNA molecule prepared according to the present invention comprising a synthetic Hu IFN-$\alpha_1$ gene and a broad host range plasmid;

Figure 14 is a schematic representation of a recombinant DNA molecule used in the present invention comprising a synthetic Hu IFN-$\alpha_1$ gene, a synthetic trp promoter, and a broad host range plasmid;

Figure 15 illustrates restriction groups of cDNA clones used in the present invention; and

Figure 16 illustrates a partial DNA sequence of a copy DNA clone used in the present invention.

Most genetic manipulation of microorganisms (i.e. modification of their genetic material, usually by insertion of foreign DNA) has involved the E. coli K12 organism or its biologically attenuated variants. E. coli, however, is found in man and there are advantages in employing an organism that is not normally found in man and which is less likely to infect him.

We have found it especially convenient to employ as the host organism an autotrophic bacterium (e.g. one capable of using as its sole or principal carbon source inorganic carbon, e.g. carbon dioxide, or a bicarbonate), or a methylotrophic bacterium (e.g. one capable of using as its sole or principal carbon source methane or 'methyl' carbon, for example from methanol). However, with the hereinbefore defined proviso, we do not exclude the possibility that the microorganism may be a fungus or alga or a bacterium which is not an autotrophe or a methylotrophe, e.g. a strain of E. coli, Salmonella, Bacillacea, e.g. B. subtilis, Pneumococcus, Streptococcus, or Saccharomyces, e.g. S. cerevisiae.

Many autotrophic bacteria are known and have been described in the literature. Examples are

EP 0 062 971 B1

*Thiobacillus* spp, *Hydrogenomonas* spp, *Chromatrium* spp, *Rhodospirillum* spp, *Nitrobacter* spp, *Rhodopseudomonas* spp, *Paracoccus* spp and *Alcaligenes* spp.

Several examples of the methylotrophic class of bacteria are known and have been described in the literature, for example *Methylophilus* spp, *Pseudomonas* spp, and *Methylomonas* spp. Specifically we may mention as examples:—

*Methylophilus methylotrophus* (AS1)
  —NC1B 10508—10515 and 10592;
  —10596, all inclusive;
  —NRRL B 5353—B 5364 inclusive;
  —FRI 1215—1227 inclusive;

*Pseudomonas methylonica*
  —the characteristics of which are described in UK Specification No. 1451020 and a culture of which is
    deposited as follows: FRI 2247 and 2248;

*Pseudomonas methanolica*
  —UK Specification No. 1352728
  —ATCC 21704;

*Pseudomonas sp*
  —UK Specification No. 1326582
  —ATCC 21438 and 21439;

*Methylomonas methanolica*
  —UK Specification No. 1420264
  —NRRL B 5458;

*Pseudomonas utilis*
  —UK Specification No. 1444072
  —FRI 1690 and 1691;

*Pseudomonas inaudita*
  —UK Specification No. 1444072
  —FRI 1693 and 1694;

*Methylomonas clara*
  —USP 4166004—ATCC 31226.

NB: NCIB is the National Collection of Industrial Bacteria, Aberdeen; NRRL is the Collection maintained by the US Department of Agriculture, Peoria, Illinois; ATCC is the American Type Culture Collection; FRI is the Fermentation Research Institute, Japan.

Typically, such micro-organisms may be aerobically cultured in an aqueous medium containing methanol as a source of assimilable carbon, together with necessary inorganic nutrients.

Of the methylotrophes, our preferred microorganism for the purpose of the invention is *Methylophilus* and specifically *M. methylotrophus*. This organism, we have found, can be grown rapidly and efficiently, its toxicology has been investigated at length, and because it does not infect man and the higher organisms it is particularly safe for large scale biosynthesis, and its genetic composition is well known.

Both anaerobic and aerobic varieties of microorganism are found, as well as both facultative and obligate organisms, and the selection of organism will be made in the light of the preferred processing conditions as well as the biochemical or biosynthetic capabilities of an organism having otherwise acceptable characteristics. In general we prefer to use an obligate organism as this may allow closer control over the process involving it.

We also provide synthetic genetic material coding for a compound having interferon activity per se or capable of being modified in vivo or in vitro to provide a compound having interferon activity and which will hereinafter be referred to for convenience as "interferon".

Genetic material coding for an interferon as hereinbefore defined will hereinafter be referred to for convenience as an "interferon gene".

As examples of interferon genes of which the microorganism prepared by the present invention may be comprised we would mention inter alia genes Hu IFN-$\alpha_1$ and Hu IFN-$\alpha_2$.

A gene of Hu IFN-$\alpha_1$ which we have prepared is based on the reported DNA sequence for Hu IFN-$\alpha_1$ (aforementioned publications by Mantei et al and Nagata et al). The gene comprises a double-stranded DNA, the coding strand of which contains the condons from the 166 amino acid sequence of Hu IFN-$\alpha_1$, preceded by an initiator codon (ATG) and the single-stranded cohesive terminus for a *Bam*HI restriction

5

endonuclease (to allow ligation at a suitable site in a plasmid vector). The structural gene is followed by a termination codon and the single-stranded cohesive terminus for a *Sal*I restriction endonuclease.

A gene for Hu IFN-$\alpha_2$ which we have prepared is based on the reported DNA sequence for Hu IFN-$\alpha_2$ (Streuli et al, Science, 1980, *209*, 1343—1347). The gene comprises a double-stranded DNA, the coding strand of which contains the codons for the 165 amino acid sequence of the Hu IFN-$\alpha_2$ preceded by an initiator codon (ATG) and the single-stranded cohesive terminus for a *Bam*HI restriction endonuclease (to allow ligation at a suitable site in a plasmid vector). The structural gene is followed by a termination codon and the single-stranded cohesive terminus *Sal*I restriction endonuclease.

The degeneracy of the genetic code permits substantial freedom in the choice of codons which can be used to construct a gene coding for the specified amino acid sequences of Hu IFN-$\alpha_1$ and Hu IFN-$\alpha_2$. The choice of codons was guided by four considerations:—

(i) the design of oligonucleotides, designated 1—67 in Figure 1 and 1—68 in Figure 2, which can be aligned, through hydrogen bonding, to the complementary strand. These oligonucleotides vary in size from 10—21 nucleotides;

(ii) the presence of self-complementarity (in five or more bases) in a particular fragment was avoided, together with the possibility of complementarity of the fragments one with another;

(iii) sequences recognised by certain restriction endonucleases were avoided, particularly if these enzymes were to be used at a latter stage; and

(iv) where changes were required as a result of consideration (i) to (iii), codons were normally chosen as those preferred in the expression of microbial genomes.

These considerations led to changes from the published nucleotide sequence, which changes are marked (.) in Figures 1 and 2; it will be appreciated that alternative termini can be used.

To facilitate synthesis of the Hu IFN genes shown in Figures 1 and 2, it is often convenient to join a few of the aforementioned oligonucleotides to form fragments (designated A—K in Example 1 and A—M in Example 8) comprising for Example 4—6 oligonucleotides. These fragments may then be assembled into sequences (designated I—IV in Example 1 and I—III in Example 8) and the sequences joined to form the genes.

However, we do not exclude the possibility that alternative synthetic strategies can be used. For example, fragments A to K have been ligated and the slowest migrating band (which had migrated a distance equivalent to a 500±20 base pair fragment) from a polyacrylamide slab or a 1.4% agarose gel was eluted to give the required gene; the mixture of products obtained in the ligation of fragments A to K, could be used in a cloning experiment, since only the intact gene, with the restriction sites at both ends, will ligate to give the circular plasmid.

Where the human interferon gene of which the microorganism is comprised is a copy gene derived from mRNA the mRNA can be isolated from a variety of cells which are known to produce human interferon. Three of the important sources of human interferon are human buffy coat cells and lymphoblastoid cells, for Hu IFN-$\alpha$, and human fibroblast cultures for Hu IFN-$\beta$. The Hu IFN's from these sources comprise a physically heterogeneous population of molecules. Preferably the mRNA is isolated from a Namalwa lymphoblastoid cell line, which is known to produce predominantly Hu IFN-$\alpha$ with a minor amount of Hu IFN-$\beta$. Production of Hu IFN's by these cells may be induced by treatment with a suitable inducer, e.g. Sendai virus.

Preferably the mRNA is at least partially enriched for IFN-coding sequences before it is used to prepare double-strand copy DNA.

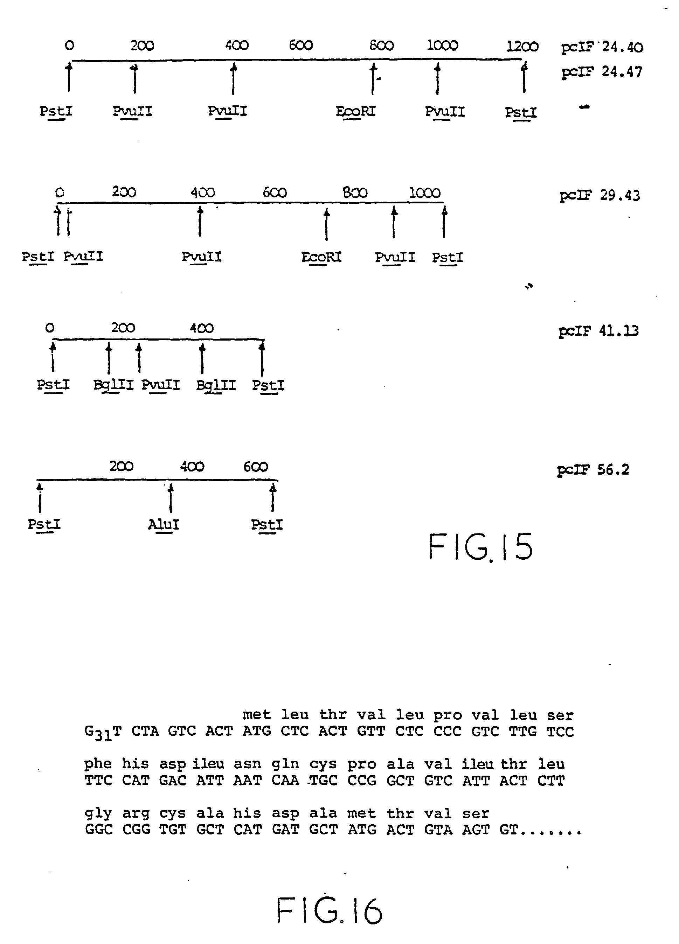

We have mapped a sample of our cDNA clones which express a metabolite having interferon activity using the restriction enzymes *Pvu*II, *Bgl*II and *Eco*RI. The results are shown in Figure 15. The restriction map of clone pcIF24.40 is similar to that of leucocyte IFN E but contains an additional *Pvu*II site in a position which corresponds to that present in the sequences of leucocyte A, D, and H (Goeddel et al, Nature, 1981, *290*, 20—26). This *Pvu*II site is in the sequence for the signal peptide of the IFN coded for by this clone.

Clone pcIF56.2 does not contain any restriction enzyme sites for the hereinbefore mentioned three restriction enzymes and we have also found that it has no restriction sites for the restriction enzymes *Hpa*II, *Bst*II, or *Taq*I. We have found a *Alu*I site. A partial DNA sequence for clone pcIF56.2 is shown in Figure 16. From Figure 16 it can be seen that there is an extensive open reading frame but there is no DNA sequence in common with any known IFN gene.

We also provide a copy DNA molecule coding for a metabolite having interferon activity per se or capable of being modified in vivo or in vitro to provide a compound having interferon activity which copy DNA molecule is derived from mRNA produced by transcription of a naturally occurring interferon gene which is a component of lymphoblastoid cells.

Methods for the introduction of foreign genetic material into bacteria have been widely described in the literature. Such methods comprise formation of recombinant DNA, which comprises a vector and the foreign genetic material, and introduction of the recombinant DNA into the microorganism.

We also provide a recombinant DNA molecule comprising (a) a synthetic interferon gene as hereinbefore defined and a suitable vector therefor or (b) a copy DNA molecule coding for a metabolite having interferon activity per se or capable of being modified in vivo or in vitro to provide a compound having interferon activity which copy DNA molecule is derived from mRNA produced by transcription of a naturally occurring interferon gene which is a component of lymphoblastoid cells and a suitable vector therefor.

Introduction of the recombinant DNA into the microorganism may be facilitated by subjecting the microorganisms to an appropriate treatment, e.g. treatment with a calcium chloride solution. Where the genetically modified organism prepared according to the present invention comprises a methylotrophe or an autotrophe the recombinant DNA comprising an interferon gene is preferably introduced thereto by transfer from another oganism by cell conjugation, more preferably by transfer of the recombinant DNA from a strain of *E. coli*.

We have found that the infection of microorganisms in the preparation of genetically modified microorganisms according to the present invention is most conveniently carried out using as vector a plasmid of one of incompatibility groups N, P, Q or W which plasmid is combined with genetic material coding for a metabolite having interferon activity to form a recombinant DNA molecule.

Plasmids of the Group Q are preferred.

The concept of incompatibility groups is discussed in "DNA Insertion Elements, Plasmids and Episomes" Ed. by Bukhari, Shapiro and Adhya, Publ. Cold Spring Harbour Laboratory 1977 at p. 601 et seq. (see also tables of plasmids in incompatibility groups at pp. 607 to 608).

While many broad host-range conjugative plasmids such as RP4 (of the Inc P group 1) can be used as the vector for cloning, they are relatively large, exist in fairly low copy number and may, therefore, not be ideal for some cloning purposes. Preferably small, high copy number, broad host range plasmids are used for cloning. R300B (of the Inc Q group) is an example of this category, but despite its advantages as a cloning vector it has relatively few sites for the restriction enzymes used in forming a recombinant DNA molecule.

It is, therefore, sometimes advantageous to construct a composite vector, by which we mean a plasmid that is not a naturally occurring plasmid, capable of both stable maintenance in the host and containing a convenient and suitable site at which foreign DNA coding for interferon may be inserted such that expression thereof in the organism may be obtained. This may conveniently be accomplished by introducing segments of DNA from other plasmids containing both a selectable marker (e.g. antibiotic resistance) and suitable restriction enzyme sites. As examples of suitable composite vectors for use in the present invention we would mention pGSS15, pGSS8 and pGSS6, which have been described in European Patent Specification No. 37273A2, and pPM50, pPM51 and pPM52, which are described hereinafter. Vectors pGSS15 pGSS8 and pGSS6 are suitable for use in E. coli and methylotrophes and autotrophes; pPM50, pPM51 and pPM52 are suitable for use in E. coli. The method of constructing such a composite vector is preferably one of broad applicability, e.g. capable of making composite vectors based on a wide range of the plasmids mentioned above and on a wide range of expressing sites, e.g. within an antibiotic resistance gene; linked to a strong promoter for the bacterium; or within a transposon.

Where the interferon gene is carried by a non-conjugative plasmid and it is desired to transfer the recombinant DNA from a first microorganism to a second microorganism the recombinant DNA may be mobilised efficiently by a suitable wide host-range conjugative plasmid, for example, from one of the aforementioned groups. For example, a wide host range mobilising plasmid, e.g. R751, may be introduced into an E. coli strain comprising a non-conjugating vector carrying an interferon gene, the strain may then be used as donors in genetic crosses with M. methylotrophus.

It is now known that prokaryotic gene expression control regions are comprised of several essential components:

(i) a transcriptional control region; consisting of a nucleotide sequence which is recognised by RNA polymerase and effects binding of the polymerase to the DNA duplex (polymerase binding site), and an adjacent sequence which triggers the polymerase to initiate production of the mRNA transcript. The whole region is generally termed the "promoter region". Overlapping the promoter region are other nucleotide sequences to which bind protein molecules that regulate the transcription process. This is the "operator" region.

(ii) a translation control region; consisting of a sequence of nucleotides present on the mRNA transcript which effects binding of the ribosomes to the RNA, probably through homology to the 3' end of the 16S ribosomal RNA, and in close proximity an ATG or GTG triplet codon (AUG or GUG on the RNA) at which initiation of translation occurs.

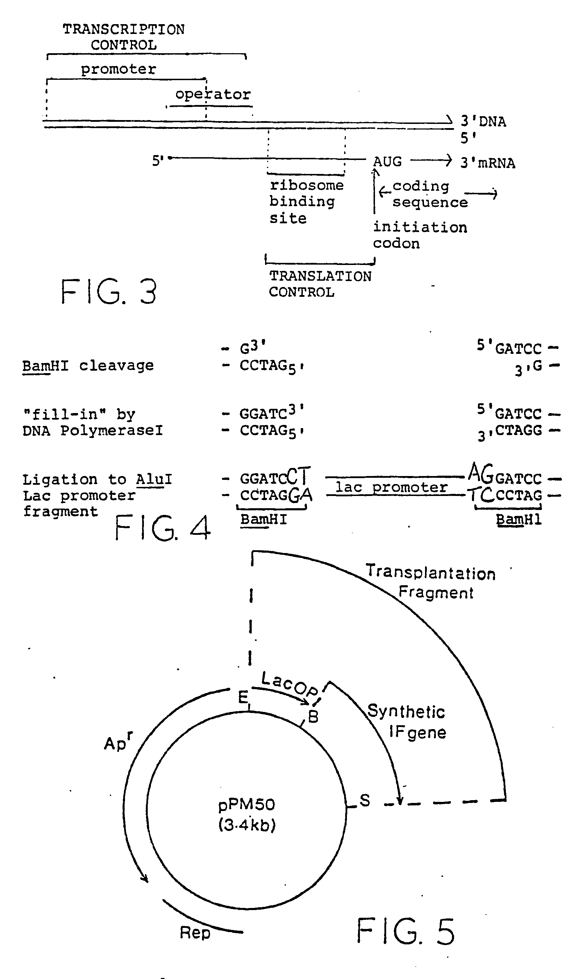

The overall structure is shown in Figure 3, although the transcriptional and translational control region need not necessarily be immediately adjacent.

Thus, it will be appreciated that in order to achieve expression the interferon gene must be positioned to lie within the transcriptional unit of a chosen promoter such that the polypeptide is produced by translation from the mRNA transcript. Depending upon the precise nature of the fusion two types of polypeptide product are possible. If the foreign DNA segment contains a complete coding sequence for the interferon gene, then insertion such that the ATG translation initiation sequence of the interferon gene is fused adjacent to a ribosome binding site will give production of a polypeptide equivalent to that for which the interferon gene codes (DNA fragments containing genes with their own ribosomes binding site, e.g. cloned prokaryotic genes, could probably be inserted at any position within the transcription unit). Alternatively, the construction can be arranged so that the fusion points occurs within the coding sequence of a prokaryotic gene and the interferon gene expressed from the chosen promoter. In this case both transcriptional and all translational control sequences are provided by the "promoter" fragment and the product is a hybrid polypeptide having the amino terminal portion of a prokaryotic protein fused to a carboxy-terminal portion consisting of the interferon gene product. Constructions of this type require detailed knowledge of the translation reading frames of the two genes in order to ensure that they are brought into phase by the fusion event, and, thus, are not preferred.

We therefore provide according to a further aspect of the present invention a method for the preparation of a recombinant DNA molecule which molecule comprises a suitable vector and a gene which encodes a compound having interferon activity per se or capable of being modified in vivo or in vitro to provide a compound having interferon activity characterised in that (i) the gene is synthesised by a process as hereinbefore described and (ii) that the gene is joined to the vector that it is under the transcriptional control of a suitable promoter and on transcription yields an mRNA molecule in which the translation initiation codon of the RNA sequence encoding the said compound is fused in the region of a ribosome-binding site such that the production of a hybrid polypeptide does not occur.

Promoters which may be used in the recombinant DNA prepared according to the present invention will be chosen with regard to the organism in which they are required to promote expression of the interferon gene. We have found that known E. coli promoters may be used to promote expression of interferon in strains of E. coli and methylotrophes, e.g. M. methylotrophus, and M. methylovora, and autotrophes, e.g. Alcaligenes eutrophus. As examples of suitable promoters we would mention *lac* and *trp* (the promoters of the E. coli lactose and tryptophan operon respectively), and mutant strains thereof, e.g. *lac UV5*, and synthetic analogues of *trp* Δ LD102.

According to a further aspect of the present invention we provide a process for the preparation of a genetically modified microorganism capable of expressing as a metabolite a compound having interferon activity per se or capable of being modified in vivo or in vitro to provide a compound having interferon activity and comprising genetic material coding for said metabolite characterised in that the process comprises the steps of (i) chemically synthesising the genetic material by the above mentioned process; (ii) synthesising a recombinant DNA molecule by the above mentioned method; and (iii) infecting the microorganism with the recombinant DNA molecule.

Genetic and biochemical studies have extensively characterised the promoter region of the *E. coli lac* operon; the complete nucleotide sequence has been determined and used to locate several mutations which affect transcriptional activity from this promoter. This has enabled the identification of the various portions of the sequence involved in RNA polymerase and *lac* repressor binding, initiation of the mRNA transcript, binding of the ribosomes to the mRNA and initiation of translation of the β-galactosidase gene. Inspection of this data shows that cleavage of *lac* operon DNA with the restriction endonuclease *AluI* (which recognises the sequence AGCT and cuts in the centre on both strands) produces a 95 bp fragment containing all features of the promoter region with the exception of the β-galactosidase ATG translation initiation codon. The *AluI* site which cuts the transcript sequence is located immediately before the ATG triplet. Therefore any gene inserted adjacent to this *AluI* site should be expressed at a similarly high level to the normal β-galactosidase.

Where this 95 bp fragment is to be used as a promoter to drive expression of an interferon gene the *AluI* site is preferably converted into a nucleotide sequence which is cleaved by restriction endonucleases which produce cohesive ended molecules. This increases the efficiency with which other DNA fragments can be ligated to this fragment, since efficiency of accurate ligation of flush-ended molecules, such as those produced by *AluI* cleavage, is much reduced as compared to that for cohesive-ended molecules.

Generation of cohesive ends was achieved by ligation of the *AluI* fragment to a plasmid pAT153 (Nature, 1980, *283*, 216—218) which had been cleaved with restriction endonuclease *EcoRI* and/or *BamHI*, and then treated with DNA Polymerase I in order to convert the cohesive ends of the molecule to flush ends. Because of the particular sequences of the *AluI* and *EcoRI* or *BamHI* cleavage sites, ligation of the *AluI* fragment to the "filled-in" vector results in regeneration of the cohesive-end restriction endonuclease site. This is shown in Figure 4 for regeneration of the *BamHI* site.

We have found two *AluI* fragments which are particularly useful as promoters for interferon genes, one of which fragments carries the UV5 mutation in the promoter which releases the transcription activity from sensitivity to catabolite repression. Derivatives of pAT153 carrying the aforementioned promoter/operator fragment were identified by use of 5 - bromo - 4 - chloro - 3 - indolyl - β - D - galactoside indicator plates which allow the identification of cells producing β-galactosidase at high level. The many copies of the pAT153 derivative cause titration of the *lac* repressor away from the chromosomal *lac* operon resulting in constitutive synthesis of β-galactosidase.

Two derivatives of pAT153 have been constructed which have the *AluI lac* promoter fragment inserted at either the *EcoRI* site (pPM52) or *BamHI* site (pPM51) so that cleavage with these enzymes generates the fragment with the appropriate cohesive ends. These fragments are therefore potentially useful as portable promoters for insertion at suitable sites into other plasmids carrying cloned genes but not expressing them. Since the fragments have identical ends 50% of the inserts should be in the correct orientation for driving expression of the cloned gene.

A third derivative (pPm50) has been made in which the *AluI* fragment is positioned between the *EcoRI* and *BamHI* sites in an orientation such that transcription is directed towards the *BamHI* site. As the fragment now has different cohesive ends it can be used as a portable promoter with directional specificity for insertion. Perhaps more usefully the pPM50 plasmid can be used as a *promoter vector* for cloning DNA fragments at the *Bam*HI site. This vector may be used for cloning interferon genes particularly the synthetic human leukocyte interferon genes hereinbefore described.

Figure 5 illustrates the structure of pPM50, the positions therein of cleavage sites for restriction endonuclease *Eco*RI (E), *Bam*HI (B) and *Sal*I (S), the location and direction of the *lac* operator/promoter region (Lac OP), and the region of the plasmid which has been used for insertion of the synthetic Hu IFN-$\alpha_1$ gene.

Where the microorganism of the present invention comprises a *trp* promoter, the promoter is preferably a deletion mutant of the E. coli *trp* promoter, e.g. a *trp* ΔLD102 (Christie et al, Journal of Molecular Biology, 1980, *143*, 335—341), which is a mutant obtained by deletion of the *trp* E and *trp* D genes, and more preferably is a synthetic analogue of such a mutant.

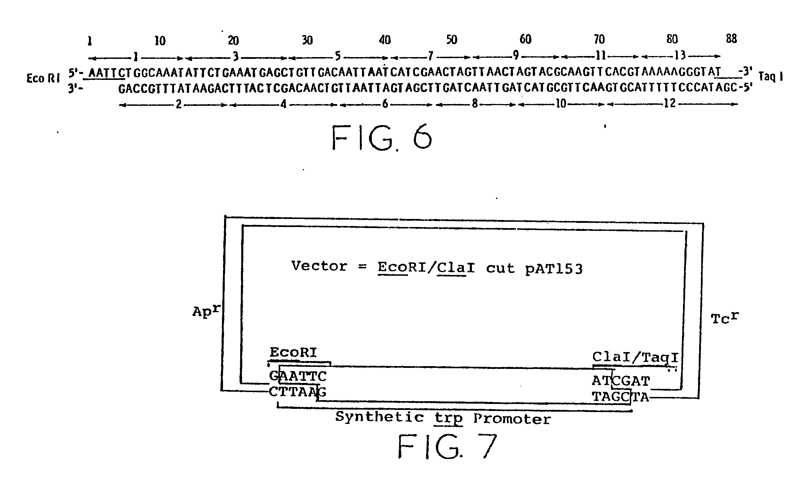

We have used a synthetic *trp* promoter fragment having the structure shown in Figure 6. It consists of the entire known essential region of the *trp* promoter (Platt in The Operon, edited by Miller and Reznikoff, Cold Spring Harbor Laboratory, 1978, pages 263—302), operator and mRNA coding sqeuences as far as the *Taq* I site downstream of the first *rbs*. For convenience a single stranded cohesive terminus for the enzyme *Eco*RI is added upstream of the *trp* promoter sequence. The cohesive single stranded end generated by the tetranucleotide recognition sequence of *Taq* I is the same as that generated by the hexanucleotide recognition sequence enzyme *Cla* I and, by choice, the flanking base pair, on the promoter proximal side of the synthetic *trp* promoter *Taq* I single stranded end (Christie et al, Journal of Molecular Biology, 1980, *143*, 335—341), is such as to regenerate a *Cla* I site on fusion to a DNA fragment cut with the latter enzyme. Plasmids such as pBR322 (Bolivar et al, Gene, 1977, *2*, pages 95—113), pAT153 (Twigg et al, Nature, 1980, *283*, pages 216—218) and pGSS15 (Barth et al in Molecular Biology, Pathogenicity and Ecology of Bacterial Plasmids, edited by Levy et al, 1980, pages 439—448) carry a single *Cla* I site 25 base pairs from their *Eco*RI site Sutcliffe, Cold Spring Harbor Symposium on Quantitative Biology, 1978, *43*, pages 77—90). They therefore provide convenient vectors for cloning the synthetic *trp* promoter fragment in such a way that it can be conveniently used to receive heterologous gene carrying DNA fragments, e.g. a gene for Hu IFN-$\alpha_1$, by insertion at the *Cla* I site (promoter vector) or to provide a source of a portable promoter (EcoRI/ClaI) fragment which can be introduced into another vector upstream of the itnerferon gene. By use of appropriate synthetic oligonucleotides, as described hereinafter, the synthetic *trp* promoter can be readily joined to the interferon gene to regenerate precisely the structure of the natural translation initiation signal of the *trp* ΔLD102 operon.

Plasmid pAT153 can conveniently be used for recombination with the synthetic *trp* promoter. It can be cleaved with *Eco*RI and *Cla*I restriction endonucleases to produce cohesive ends capable of hybridising with and being joined to the EcoRI and TaqI cohesive ends of the synthetic *trp* fragment as shown in Figure 7.

Two *E. coli* clones which contained plasmids with the anticipated size of insert to be carrying the synthetic *trp* promoter fragment were obtained from a ligation mixture containing *Eco*RI/*Cla*I digested pAT153 and the synthetic *trp* promoter fragment. These plasmids had single *Eco*RI and *Cla*I sites and cleavage with both enzymes produced approximately 88 base pair fragments, whereas cleavage of the parental pAT153 plasmid with these enzymes produced a 24 base pair fragment.

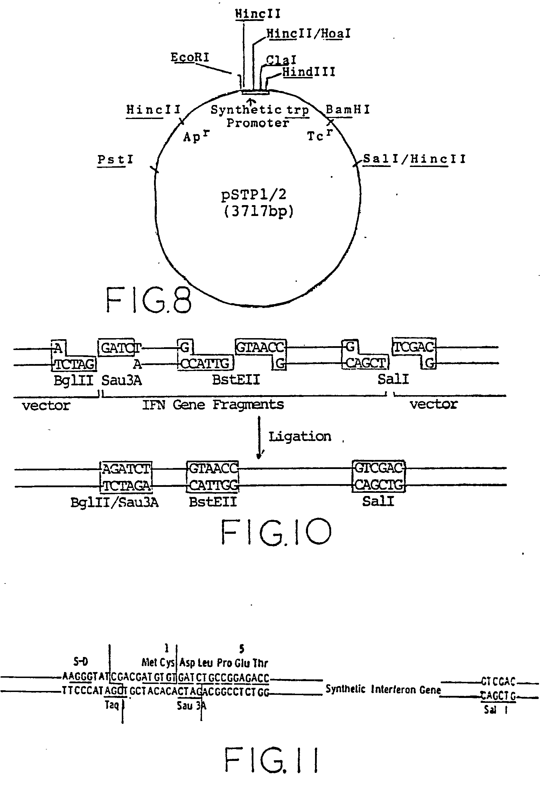

These two plasmids have been assigned the designations pSTP1 and 2. The structure of pSTP1 and pSTP2 is shown in Figure 8. They may be used both as sources of a "portable *trp* promoter" which can readily be transplanted into other plasmids and as "promoter vectors" to allow foreign genes to be introduced into the *Cla*I site just downstream of a potentially very powerful promoter.

In the synthetic Hu IFN-$\alpha_1$ gene shown in Figure 1 there is a *Bam*HI restriction enzyme site at the position in which a *Cla*I site is desirable for joining with the synthetic *trp* promoter. Also the nucleotide sequence between the *Bam*HI site and the translation initiation codon for the IFN gene is not the same as that of the corresponding region of the *trp* operon in the *trp* ΔLD102 deletion, which is known to result in a very high efficiency translation initiation sequence and was therefore one of the sought after features in using the *trp* promoter expression system.

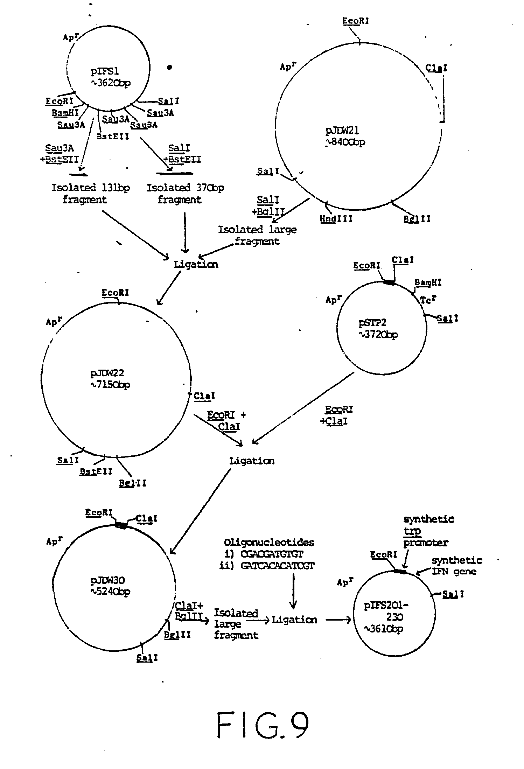

It will be appreciated that where it is desired to combine the synthetic *trp* promoter with the Hu IFN-$\alpha_1$ gene shown in Figure 1, it is necessary to modify the promoter and/or the gene to effect recombination thereof. Figure 9 illustrates, in outline, a series of manipulations by which such recombination can be effected; it will be readily apparent to the skilled man that various alternative modifications can also be employed.

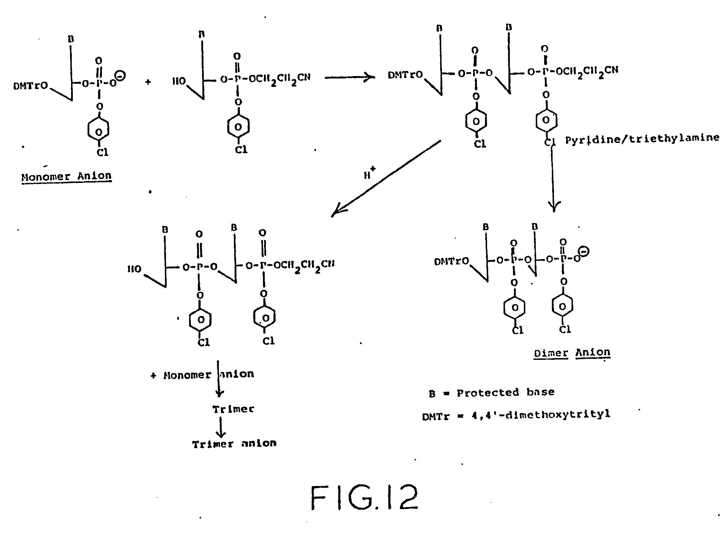

The synthetic Hu IFN-$\alpha_1$ gene was separated, as two fragments, from plasmid pIFS1 (as hereinafter described) and recombined with vector pJDW21 (pJDW21 was constructed from pAT153 by insertion of an *Eco*RI/*Hind*III fragment of a derivative of bacteriophage λJW19). Details of the recombination to form pJDW22 are shown in Figure 10. A fragment of pJDW22 containing the synthetic Hu IFN-$\alpha_1$ gene was combined with a fragment of pSTP2 containing the synthetic *trp* promoter to form pJDW30. Modification of pJDW30 as shown in Figure 9 gave desired recombinant DNA molecules pIFS201—230 which have the synthetic *trp* promoter and synthetic Hu IFN-$\alpha_1$ gene in the correct orientation for transcription of the gene.

Naturally produced (i.e. by the living cell) interferon is thought to comprise at least a protein component and a sugar-based component. It is likely that the desirable interferon activity is located primarily in the protein component, perhaps modified, for example in its specificity or otherwise by

9

non-protein components. The interferon gene employed according to the present invention codes specifically for a metabolite which may itself have interferon activity, or may be a precursor for a compound having interferon activity, for example after modification, either in vivo or in vitro, to promote, enhance or modify its activity. In vitro modification may involve the use of biochemical techniques for example emulating those in the living cell, although more conventional chemical techniques are not excluded.

The modification of interferons has been described for example in UK Patent Specification Nos. 1471461, 1471462 and 1471463.

We have found that a metabolite which is expressed by a synthetic interferon gene of the present invention under control of a *lac* UV5 promoter, which metabolite has interferon activity, has a molecular weight of about 16000—17000. Conventional Hu IFN-$\alpha_1$ has a molecular weight of approximately 19000. Without the intention of being limited by the following explanation we believe that about 18—20 amino acids, probably the first 18—20 amino acids, of naturally occuring Hu IFN-$\alpha_1$ are missing.

We therefore also provide a polypeptide of molecular weight about 16000—17000 which exhibits IFN activity.

A genetically modified organism prepared according to the present invention may be employed in a variety of applications, depending, of course, upon its physiology and upon the nature of the inserted DNA.

Most conveniently, it will be used in a biosynthetic mode, in a suitable nutrient medium, metabolite preferably being passed out of the cell into the surrounding culture solution and extracted therefrom in the conventional way. Alternatively, where, for example, the metabolite is not passed out of the cell at a commercially useful rate, the organism may be cultured and harvested as the intact cell conveniently with subsequent extraction of the cells for example, after separation from the medium. Such techniques are well known and require no further explanation.

The interferon expressed may be used as obtained, it may be purified in known or appropriate manner, and it may be mixed with suitable inert or appropriately active diluents, which typically will be liquid but may be solid, to produce a pharmacologically acceptable composition.

Interferons of the present invention are potentially useful as antiviral, or anticancer agents or as modulators in the immune response of, for example, mammals and particularly man.

Various aspects of the present invention will now be described with reference to the following Examples which are illustrative of the invention.

Example 1

This example illustrates the preparation of a Hu IFN-$\alpha_1$ gene, formation of a recombinant DNA molecule comprising the gene and a *lac* promoter and expression of the gene in *E. coli*.

Synthesis of dimer anions

Fully protected nucleoside and oligonucleotide phosphotriester building blocks were prepared essentially as previously described by J. Stawinski et al., Nucleic Acids Research, 4, 353—371 (1977), or Katagiri et al, J. Amer. Chem. Soc., 97, 7332—7337 (1975), and T. Hirose et al., Tetrahedron Letters, 2449 (1978), according to the reaction sequence shown in Figure 12. The benzoyl protecting group was used with cytidine residues. The dimer blocks were purified by high pressure liquid chromatography (HPLC) on a Waters Prep. 500, using a gradient of methylene chloride to 6,8 or 10% methanol in methylene chloride.

Removal of cyanoethyl protecting groups followed the procedure of Sood and Narang (Nucleic Acids Research, 4, 2757—2765 (1977)), except that the phosphodiester products were freed from cyanoethanol by partition between chloroform and saturated brine. The organic phase was evaporated, and the products were precipitated from pyridine solution with diethyl ether/n-pentane (3/2).

Oligonucleotide assembly on the polyacrylamide solid phase, cleavage from the resin and removal of protecting groups, were as described, using a bench-top manual solvent delivery system in M. J. Gait et. al., Nucleic Acids Research 8, 1081—1096 (1980); and A. F. Markham et. al., Nucleic Acids Research 8, 5193—5205 (1980). The intermediate removal of the 5′ - dimethoxytrityl group was carried out by 2×5 second treatment with 5% benzenesulphonic acid in chloroform/methanol (7/3). The coupling reaction was carried out for one and a half hours in the presence of the coupling agent (2,4,6 - tri - isopropylbenzene-sulphonyl tetrazole or 1 - (mesitylene - 2 - sulphonyl) - 3 - nitro - 1,2,4 - triazole). The resin was dried by azeotropic evaporation from anhydrous pyridine (1 ml) prior to the coupling reaction.

Synthesis and purification of oligodeoxynucleotides

Sixty seven oligodeoxynucleotides designated 1 to 67, as indicated by the arrows above and below the oligonucleotide sequence in Figure 1, were synthesised by a solid phase procedure (from a library of dimers) on a polyacrylamide support (Markham et al., Nucleic Acid Research, 1980, 8, 5193—5205; Gait et al., Nucleic Acids Research, 1980, 8, 1081—1096). Functionalised polydimethylacrylamide resin (100 mg), substituted with the appropriate 5′ - O - dimethoxytrityl - 2′ - deoxynucleotide via a 3′ - O - succinamido linkage (approx. 26 µmol total functionality) was swollen in dimethylformamide then treated with phenylisocyanate as described in Markham et al. Each oligonucleotide assembly involved addition of protected dinucleotide (or mononucleotide) units by the following cycle. The resin was washed sequentially with pyridine (×5); chloroform/methanol (7/3, ×6); 5% benzenesulphonic acid in chloroform/methanol (7/3, for 2×5 secs to remove dimethoxytrityl group); dimethylformamide (×4); pyridine (×8).

Each washing operation involved a solvent volume of 6 ml. The resin was dried by co-evaporation with anhydrous pyridine (1 ml) and the dinucleotide anion (100 mg, approx. 3 equivalents previously dried by pyridine co-evaporation) was added in anhydrous pyridine (4 ml) followed by the coupling agent, mesitylene - 3 - nitro - 1,2,4 - triazole (60 mg, approximately 8 equivalents) or 2,4,6 - trisopropylbenzene-sulphonyl tetrazole (70 mg, approx. 8 equivalents). The coupling reaction was allowed to proceed, with shaking, for 1—1.5 hours. The cycle was repeated with the appropriate dinucleotide anions until the required sequence was obtained. The resin was finally washed (5 volumes each) with dimethylformamide, methylene chloride diethyl ether, then dried in vacuo. (Reactions can be carried out on half the scale described, using 50 mg of functionalised resin and 50 mg of dinucleotide anion). Each oligonucleotide was cleaved from the solid phase (40 mg of oligomer-substituted resin) and all protecting groups were removed (procedure B, Gait et al., Nucleic Acid Research, 1980, 8, 1081—1096) by sequentially reacting with 0.3M 1,1,4,4 - tetramethylguanidinium 4 - nitrobenzaldoximate in dioxan/water (1/1, 2 ml) at room temperature for 60 hours; concentrated ammonia at 50°C for 5 hours; 80% aqueous acetic acid for 30 minutes at room temperature. The deprotected oligomer was dissolved in pyridine (0.05 ml), ethanol/water (3/7, 2—4 ml) and suitable aliquots were purified initially by HPLC on Partisil 10-SAX.

Columns were eluted at a flow rate of 2 ml min$^{-1}$ with a gradient from 1 mM to 0,4 M potassium phosphate, pH 6.8 in 5% ethanol or pH 6.5 in 30% ethanol, over a period of 50 minutes at ambient temperature. The most retained peak(s) were collected, evaporated, redissolved in water (5 ml) and applied to a μ-Bondapak C$_{18}$ reverse-phase column. The oligonucleotide was further purified by first eluting with 0.1 M ammonium acetate, then with a gradient of 10—25% 0.1M ammonium acetate/acetonitrile (1/1) over 40 minutes with a flow rate of 2 ml min$^{-1}$ at ambient temperature. After addition of excess triethylamine, oligonucleotide-containing fractions were lyophilized and redissolved in water (1 ml). Aliquots of each solution (80 pmol of oligonucleotide) were evaporated and phosphorylated with 1.1 units of T4-induced polynucleotide kinase in 10 μl of a solution containing 160 pmol [γ-$^{32}$P]ATP (12.5 Cimmol$^{-1}$), 0.1 mM spermidine, 20 mM dithiothreitol (DTT), 10 mM MgCl$_2$, 50 mM Tris-HCl (pH 9.0) and 0.1 mM EDTA for 45 minutes at 37°C. Reactions were terminated by addition of 270 μl of 50 mM Tris-borate (pH 8.3), 1 mM EDTA in 80% formamide and heating to 90°C for 5 minutes. A 7 μl aliquot was examined on a 20% polyacrylamide gel in 7 M urea.

Ligation of chemically synthesised oligonucleotides

(a) Ligation strategy. Aliquots of the chemically synthesised oligonucleotides, 2 to 65 and 67 (400 pmole of each), were phosphorylated with 6.5 units of T4-induced polynucleotide kinase in 25 μl of a solution containing 800 pmol [γ-$^{32}$P]ATP (8.5 Cimmol$^{-1}$), 100 μM spermidine, 20 mMDTT, 10 mM MgCl$_2$, 50 mM Tris-HCl (pH 9.0) and 0.1 mM EDTA for 30 minutes at 37°C. Each oligonucleotide was ethanol precipitated and fractionated by electrophoresis on a 20% polyacrylamide gel in 7 M urea. The $^{32}$P-labelled oligomers were sliced from the gel and eluted. Eleven separate T4 ligase-catalysed joining reactions were performed to give the double-stranded fragments A to K shown in Table 1.

TABLE 1

| Fragments | Oligonucleotides | Number of bases in | |
|---|---|---|---|
| | | Top strand | Bottom strand |
| A | 1—5 | 44 | 32 |
| B | 6—11 | 45 | 47 |
| C | 12—17 | 46 | 44 |
| D | 18—23 | 45 | 46 |
| E | 24—29 | 45 | 45 |
| F | 30—37 | 60 | 59 |
| G | 38—43 | 45 | 45 |
| H | 44—49 | 47 | 47 |
| I | 50—56 | 45 | 60 |
| J | 57—61 | 46 | 30 |
| K | 62—67 | 42 | 55 |

11

In Fragments A and K, oligonucleotides 1 and 66, respectively, were not phosphorylated. For the construction of each fragment, the terminal oligonucleotides were present in excess over the internal oligonucleotides. For example, fragment B was constructed by annealing, in 50 µl water, 38 pmol of oligonucleotides 6 and 11, 36 pmol of 7 and 10 and 34 pmol of 8 and 9, heating to 100°C and cooling slowly to room temperature. Each mixture was dried in vacuo and ligated in 20 µl of a solution containing T4 DNA ligase (26 units), 74 mM Tris-HCl (pH 7.6), 6.6 mM MgCl$_2$, 10 mM DTT, 2.9 mM ATP, 1.6 mM mercaptoethanol and 1.6 µg bovine serum albumin for 24 hours at 22°C.

(b) Aliquots (1%) of the reaction mixtures were examined on a 15% polyacrylamide gel in 7 M urea.

(c) The DNA from each of the remaining mixtures was ethanol precipitated and fractionated on a 15% polyacrylamide gel in the absence of urea. The DNA from each band was eluted from the gel. An aliquot of each band was re-run in denaturing conditions. In each group, the most slowly migrating band had oligonucleotide strands of the correct size.

(d) Sequences (I—IV) were constructed from 1.0 pmol of fragments A—K as shown in Table 2.

TABLE 2

| Sequences | Fragments | Number of bases in | |
| --- | --- | --- | --- |
| | | Top strand | Bottom strand |
| I | A+B+C | 135 | 123 |
| II | D+E | 90 | 91 |
| III | F+G+H | 152 | 151 |
| IV | I+J+K | 133 | 145 |

Annealing was carried out at 45°C and the mixtures were ligated as hereinbefore described. The fragments were isolated by electrophoresis on a 10% polyacrylamide gel in the absence of urea.

(e) The ligation of sequences I—IV (0.1 pmol of each), to give the complete gene, was carried out as previously described for the preparation of sequences I—IV, to give the product, at approx. 500 bp.

A first sample of the product after precipitation was purified by two procedures (a) on a 3.5% polyacrylamide slab, or (b) on a 1.4% agarose slab. In both cases, the band corresponding to 514 base pairs was eluted from the gel, ethanol precipitated and phosphorylated with polynucleotide kinase (hereinafter referred to for convenience as SIF Gene A). A second sample of the product after precipitation was phosphorylated with polynucleotide kinase (hereinafter referred to for convenience as SIF Gene B).

Construction of composite vector

Vector plasmid pAT153 (7 µg) (Twigg et al., Nature 1980, *283*, pages 216—218) was digested sequentially for 60 minutes at 37°C with restriction endonucleases *Eco*RI and *Bam*HI in 10 mM Tris-HCl (pH 7.6), 6 mM MgCl$_2$ and 1 mM DTT containing 50 mM (Eco RI) or 150 mM (*Bam*HI) NaCl. Reactions were terminated by extraction with phenol/chloroform (3/1) and ethanol precipitation. The DNA was resuspended in 30 µl DNA polymerase I buffer (6 mM Tris-HCl (pH 7.4), 7 mM MgCl$_2$, 1 mM DTT) containing 40 µM each of dATP, dGTP, dCTP, and dTTP to synthesise 3'-oligonucleotide extensions complementary to the protruding 5'-oligonucleotide extension generated by the endonuclease treatment. The reaction was initiated by addition of DNA polymerase I-Klenow fragment (1.5 units) and incubated at 11°C for 90 minutes. $^3$H-dTTP (3 µCi mmol) was added to follow the extent of reaction by trichloroacetic acid precipitation of 5-µl samples. The reaction was terminated by extraction with phenol/chloroform (3/1) and ethanol precipitation. Plasmid pPM18 is a derivative of plasmid pACYC184 (Chang et al, J. Bact., 1978, *134* pages 1141—1156) which has a 6.6 kb *Eco*RI fragment derived from λplac 5 (Shapiro et al. Nature 1979, *224*, 768—774) inserted at the *Eco*RI site in the plasmid chloramphenicol resistance gene. The λplac5 fragment carries the promoter region of the E. coli lactose operon and part of the β-galactosidase gene. Plasmid pPM18 (15 µg) was digested with restriction endonuclease *Alu*I in 6 mM Tris-HCl (pH 7.6), 6 mM MgCl$_2$, 50 mM NaCl, 6 mM β-mercaptoethanol and the 95 bp fragment containing the E. coli lactose operon/promoter region isolated by electroelution from a 5% polyacrylamide gel and purified by chromatography on DE52DEAE-cellulose. The "filled-in" *Eco*RI/*Bam*HI cleaved pAT153 was blunt-end ligated to the 69-bp *Alu*I fragment in a volume of 50 µl in 20 mM Tris-HCl (pH 7.6), 10 mM MgCl$_2$, 10 mM DTT, 5 mM ATP, with T4 DNA ligase (5 units) at 10°C for 16 hours. The ligated mixture was transformed to E. coli strain C600 (Bachmann, Bact. Rev., 1972, *36*, pages 525—557) and ampicillin-resistant transformant colonies selected on antibiotic (25 µg ml$^{-1}$) minimal X-gel (20 µg ml$^{-1}$) medium. Colonies containing recombinant plasmids with the *lac* promoter/operator sequence were identified by their blue colouration on the X-gel medim, due to constitutive expression of the chromosomal β-galactosidase gene. Plasmid DNA isolated from eight independent blue colony transformants, was screened by digestion with restriction endonuclease *Eco*RI

# EP 0 062 971 B1

and *Bam*HI and one found to contain a single cleavage site for each enzyme separated by approximately 100 bp. Nucleotide sequence determination of the 100 bp fragment showed that it contained the *lac* promoter/operator sequence flanked by *Eco*RI and *Bam*HI sites and orientated such that transcription would proceed towards the *Bam*HI site. This plasmid pPM50 was used as a composite vector for the subsequent cloning of synthetic interferon genes.

## Construction of recombinant DNA

pPM50 (4 µg) was digested with restriction endonucleases *Bam*HI and *Sal*I in 10 mM Tris-HCl (pH 7.6), 6 mM $MgCl_2$, 150 mM NaCl and 1 mM DTT at 37°C for 60 mins. The reaction was terminated by extraction with phenol/chloroform and the products subjected to electrophoresis through 1% agarose in 40 mM Tris-HCl (pH 7.8), 6 mM sodium acetate, 1 mM EDTA buffer. A 3.2 kb fragment, the larger one present, was isolated by electroelution and chromatography on DE52 DEAE-cellulose. The purified 3.2 kb fragment (1 µg) was ligated in separate experiments to the synthetic interferon genes SIF Gene A and SIF Gene B with T4 DNA ligase (0.4 units) in a total reaction volume of 30 µl containing 20 mM Tris-HCl (pH 7.6), 10 mM $MgCl_2$, 10 mM DTT at 12.5°C for 16 hours. The reaction mixtures were separately used to transform competent cells of E. coli K-12.

## Transformation of E. coli K-12 with recombinant DNA

Cells of E. coli K-12, strain MRC 8 (F, *dap* 103, *hsd* R, *met* B1, *glm* 533, *upp* 1, *dap* 101, *sup* E, *rec* A), were rendered competent by treatment with calcium as follows: a fresh overnight culture of the recipient strain was diluted 1:5 in LB and grown at 37°C with vigorous aeration, for approximately 100 min, then cooled rapidly on ice. The cells were pelleted by centrifugation at 4,000 rpm for 10 min and resuspended in 0.5 volume of ice cold 10 mM calcium chloride. After pelleting once more the cells were resuspended in 0.05 volume of 75 mM calcium chloride and kept on ice.

200 µl of these competent cells were mixed with 0.1—1 µg of recombinant DNA contained in the reaction mixture described in the section "Construction of Recombinant DNA" in 100 µl of SSC (150 mM sodium chloride, 15 mM tri-sodium citrate) and incubated on ice for 30 mins. The mixture was heated at 42°C for 2 mins, followed by a further 20 mins on ice. The cells were then diluted 1:10 in LB and incubated at 37°C for 45—60 mins, to allow expression of plasmid encoded functions, then were concentrated by centrifugation and resuspended in 100 µl of bacterial buffer ($KH_2PO_4$, 3 g; $Na_2HPO_4$, 7 g; NaCl, 4 g; $MgSO_4 . 7H_2O$ 0.2 g per litre) before being plated out on selective L-agar media containing 50 µg/ml ampicillin, N-acetyl glucosamine (0.02%) and diaminopimelic acid (50 µg $ml^{-1}$). This procedure typically produced $10^4$—$10^6$ antibiotic resistance colonies per µg of recombinant DNA. Transformant colonies containing recombinant DNA which comprised the synthetic interferon gene were identified by Grunstein-Hogness colony hybridisation using a radioactivity $^{32}$P-labelled oligonucleotide from within the synthetic sequence (coding for amino acids 78—97) by hybridisation probe. Two positively responding colonies were detected among the transformants arising from the ligation of the 3.2 kb vector plasmid to SIF Gene A and a further 12 positively responding colonies were detected among the transformants arising from the ligation of the 3.2 kb vector plasmid to SIF Gene B.

Analysis of the plasmid DNA isolated from these colonies showed that all 14 contained equivalent-size plasmids (hereinafter referred to for convenience as pIFS1-14). Four of these plasmids were selected for more detailed analysis (pIFS 1, 2, 4, 14). Restriction enzyme analysis of pIFS1, 2, 4 and 14 and nucleotide sequence analysis of pIFS1 confirmed that the structure of these recombinant DNA molecules was in agreement with the theoretical sequence shown in Figure 1.

Positive clones were grown in Luria broth containing tetracycline (10 µg/ml) or ampicillin (25 µg/ml). Overnight cultures were inoculated into fresh medium and grown to early stationary phase (approx. $1 \times 10^9$ cells/ml). The cells were harvested and resuspended in 0.01 volumes of 50 mM Tris-HCl pH 8.0, 50 mM EDTA, 15% sucrose. Lysozyme was added to a final concentration of 2.5 mg/ml and the suspension kept on ice for 30 mins before freezing at −20°C. The frozen lysate was thawed at room temperature and sonicated for 15 sec. The sonicated lysate was aliquoted and stored at −20°C or below until thawed for bioassay.

## Interferon bioassay

Serial two fold dilutions were made in 96 well microtitre plates (Falcon plastics). T98G cells (a human neuroblastoma cell line), MDBK (bovine) cells, or L cells (murine) were added to each well and the cultures incubated overnight at 37°C in a $CO_2$ incubator. T98G cultures were challenged with encephalomyocarditis virus and MDBK cultures with vesicular stomatitis virus (0.5 PFU/cell) and scored for cytopathic effect 24—48 hours later. The titre of a sample (expressed as units/0.15 ml bacterial extract) was defined as the reciprocal of the maximum dilution reducing the cytopathic effect by 50% or more. In this assay the NIH human reference standard G-023-901-527 (20,000 Iu/ml) diluted 1 in 50 and containing by definition 400 Iu/ml gave a titre of 120 units/0.15 ml in T98G cells. The MDBK cell line is approximately 20-fold more sensitive than the T98G cells for the IFN-$\alpha_1$ species (see Table 3).

## Antiserum neutralisation

This was performed in an interferon assay as described above. For each serum tested, 10 µl of the appropriate dilution was added to two rows of the microtitre plate.

13

In order to determine whether an interferon-like polypeptide is produced, we have assayed extracts of *E. coli* cells containing the pIFS plasmids for the production of an antiviral activity. The results, presented in Table 3 for two of these clones, clearly show that a significant level of antiviral activity was detected in cells containing the recombinant plasmids. No such activity was found in cells containing only the vector plasmid pPM50 (pAT153 carrying the wild type *lac* promoter).

Since the nucleotide sequence of the chemically synthesised interferon gene was altered from that of the natural gene, as defined by the sequence of the $\alpha_1$ cDNA (Nagata et al. as hereinbefore mentioned), it was important to establish that the polypeptide which we had prepared produced antiviral activity which was substantially indistinguishable in at least many tests from that produced in *E. coli* cells expressing $\alpha_1$ cDNA (Stewart et al. as hereinbefore mentioned). The following properties have therefore been investigated:

(i) Stability at pH2. Stability at pH2 was one of the first described characteristeics of IFN-α and β (Isaacs et al, Proceedings Royal Society B, 1957, *147*, 268—273) and we tested whether the synthetic IFN-α1 gene product displayed this property. For this purpose, 10 μl of the bacterial extract were diluted either in Sorensen buffer at pH2, or in phosphate buffered saline (PBS) at pH7.4 and kept at 4°C for 24 hours, after which time the antiviral activity was determined. Table 3 shows that, like natural interferon, the synthetic gene product is acid stable.

(ii) Neutralisation of the antiviral activity. The antiviral activity was further characterised in a neutralisation assay in the presence of five different anti-interferon sera. The results in Table 3 show that the activity of the product from the two clones tested was neutralised by the anti-Namalwa IFN serum and two different anti-IFNα sera but not by two different anti-IFNβ sera. This demonstrates that the antiviral activity can be correlated with the synthesis of a compound having the biological activity of an IFNα.

(iii) Antiviral activity on bovine and murine cells. The effect of various human interferons on heterologous cells has been extensively documented. The bacterially derived synthetic gene product was assayed on human, bovine and murine cells. The spectrum of activity is shown in Table 3 and corresponds in profile to that reported for the IFN-α1 produced by E. coli containing α1 cDNA (Stewart et al. as hereinbefore mentioned).

### TABLE 3
### Characterisation of antiviral activity of E. coli containing pIFS plasmids*

| | Stability to acid pH[+] | | Neutralisation by antisera[+] | | | | Activity on cells of different species | | |
|---|---|---|---|---|---|---|---|---|---|
| Plasmid | pH 7.4 | pH 2.0 | No serum | Anti Namalwa IFN | Anti-leucocyte IFN | Anti-fibroblast IFN | T985G Cells (human) | L cells (murine) | MDBK Cells (bovine) |
| pIFS1 | 520 | 1200 | 1200 | <75 | <75 | 1200 | 1200 | 600 | 25,600 |
| pIFS11 | 1040 | 1040 | 1200 | <75 | <75 | 1200 | — | — | — |

\* Expressed as antiviral units/0.15 ml cell extract.
[+] Assayed on T98G cells.

Stimulation of 2',5' - oligoadenylate synthetase activity

Interferon treatment of cells has been shown to stimulate the activity of the enzyme 2',5'-oligoadenylate synthetase (Williams and Reed in Biology of the Interferon System, Edited by De Maeyer et al, Elsevier, Holland, 1981, pages 111—114).

Example 2

This example describes the preparation of a recombinant DNA molecule comprising the gene prepared in Example 1 and a *lac* UV5 promoter (Mercereau-Puijalan, Nature, 1978, *275*, pages 505—510) and expression of the gene in *E. coli*.

The procedure for the preparation of a recombinant DNA molecule described in Example 1 was repeated except that the *lac* UV5 promoter was used instead of the wild-type *lac* promoter. The recombinant DNA molecules prepared were assigned the designations pIFS101—108. The expression of interferon from a sample of these recombinant DNA molecules, namely pIFS101, was shown by the bioassay method described in Example 1. The antiviral activity expressed as antiviral units/0.15 ml bacterial extract as assayed on MDBK cells was $1.3 \times 10^6$. The molecular weight of pIFS101 determined by SDS polyacrylamide gel electrophoresis was about 16000—17000.

Examples 3—6

These examples describe transfer of the synthetic gene prepared in Examples 1 and 2 from E. coli to other organisms and production therein of interferon.

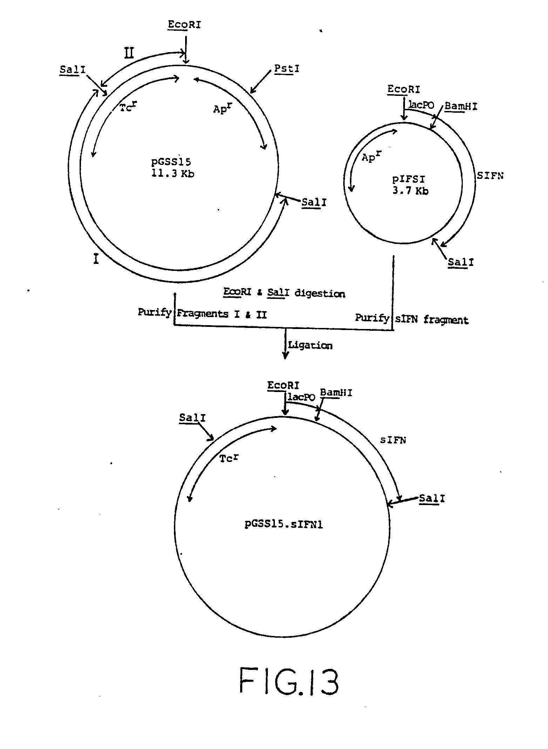

The DNA sequences comprising synthetic interferon gene/promoter prepared in Examples 1 and 2 were recloned into plasmid pGSS15 (Barth et al, Molecular Biology, Pathogenicity and Ecology of Bacterial Plasmids, Eds. Levy SB, Clowes, R. C. and Koenig, E. L. (Plenum Publishing Corporation, New York) as shown in Figure 13. pGSS15 was digested to completion with *Eco*RI and *Sal*I and the digest products separated by agarose gel electrophoresis. The synthetic gene promoter fragments were obtained from an *Eco*RI and *Sal*I digeste of pIFS1 and pIFS105 separately and were separately ligated to the replicon (I) and Tc$^r$(II) bands purified from pGSS15. After transformation of E. coli, hybrids were selected as being Tc$^r$, Ap$^s$, giving a positive Xg reaction (Davies, J and Jacob, F, J. Mol. Biol., *36*, 413—417) indicating the presence of the lac promoter sequence and hybridising to a nick translated 60 base pair fragment from the synthetic gene sequence.

A wide host range mobilising plasmid, R751 (Bennett et al. Journal of Bacteriology, 1976, *126*, pages 1—6) was introduced into E. coli PA34OrK$^-$mK$^+$ carrying the pGSS15-sIFN recombinants. These were then used as donors in genetic crosses with a range of recipient hosts as described previously for M. methylotrophus (Windass et al., Nature, 1980, *287*, 396—401). The host/recombinant DNA combinations used are shown in Table 4. The genetically modified microorganisms express material having interferon activity.

TABLE 4

| Example No. | Recipient host | Transformant |
|---|---|---|
| 3 | Methylomonas methylotrophus | pGSS15—IFS1 |
| 4 | Pseudomonas methylonica UK specification No. 1451020 | pGSS15—IFS105 |
| 5 | Methylomonas methylonica UK Specification No. 1420264 | pGSS15—IFS105 |
| 6 | Alcaligenes eutropha ATCC 17698 | pGSS15—IFS105 |

Example 7

This example describes the formation of a recombinant DNA molecule comprising the gene prepared in Example 1 and a synthetic *trp* promoter and expression of the gene in E. coli and M. methylotrophus. The synthetic promoter sequence is shown in Figure 6, and includes all the known control regions to achieve expression of a gene, containing an initiation codon at a suitable distance downstream. The sequence comprises a double-stranded DNA, preceded by a single-stranded cohesive terminus for an *Eco*RI

EP 0 062 971 B1

restriction endonuclease and terminated by a single-stranded cohesive terminus for either a *Taq*I or *Cla*I restriction endonuclease.

Thirteen oligodeoxynucleotides designated 1—13, as indicated by the arrows above and below the oligonucleotide sequence of Figure 6 were prepared as described in Example 1, except that the solvent used for hplc ion-exchange chromatography was phosphate buffer (pH 6.0) containing 30% formamide.

Ligation of fragments 1—5 to form fragment I

The 5'-hydroxy termini of the chemically synthesised oligonucleotides 2 to 5 shown in Figure 6 (0.4 n mol) were separately phosphorylated with T4 polynucleotide kinase as described in Example 1, to a specific activity of 8.4 Ci mmol$^{-1}$. Each oligonucleotide was precipitated with ethanol and subjected to electrophoresis on a 20% polyacrylamide gel containing 7 M urea. The $^{32}$P-labelled oligonucleotides were detected by autoradiography, excised from the gel and electroeluted in 1.5 ml of 10 mM Tris-borate (pH 8.3), 0.2 mM EDTA by the method described by McDonnell et al, Journal of Molecular Biology (1977), *110*, 119—146, except that the dialysis bags were clipped rather than tied, the electrophoretic polarity was reversed for 15 seconds at the end of the electroelution and the solutions were not extracted with phenol.

· The eluates were chromatographed on DE52 cellulose. After electroelution, the electrophoretic buffer was applied to 75 µl of DE-52 cellulose contained in a 1 ml Gelson pipette tip which had been plugged with siliconised glass wool. The eluate from this column was passaged 3 times through the column to ensure all the oligonucleotide fragment had been absorbed. After washing with water (1 ml) the oligonucleotide was eluted with 3 100 µl volumes of 2 M sodium chloride containing 50 mM Tris-HCl (pH 7.5) 1 mM EDTA and precipitated by addition of ethanol (1 ml) and cooling to −70°C for 16 hours.

As an alternative to the DE52 chromatography the eluate in the dialysis bag was extracted with butanol to a volume of approximately 20 µl. Water (160 µl) and 3 M sodium acetate, pH 5.6 (20 µl) were added with mixing then the oligonucleotide was precipitated by addition of ethanol (1 ml) and cooling at −70°C for at least 1 hour. The products were Cerenkov counted, aliquots of $^{32}$P-labelled oligonucleotides 2 to 5 (64, 60, 56 and 60 pmol, respectively) were combined and dried *in vacuo*. The mixture was dissolved in sterile distilled water (50 µl) and heated to 100°C for 5 minutes. The solution was allowed to cool slowly to room temperature, lyophilised and ligated with T4 DNA ligase (26 units) in 20 µl containing 76 mM Tris HCl (pH 7.6), 6.6 mM magnesium chloride, 10 mM dithiothreitol, 2.9 mM ATP, 1.6 mM mercaptoethanol and 1.6 µg bovine serum albumin. The reaction temperature was initially 37°C for 30 minutes, then reduced to 18°C and maintained at this temperature for 5 hours. The temperature was further reduced to 7°C over 4 hours then increased to 22°C for 5 hours. 0.3 M sodium acetate (250 µl) was added and the product was precipitated by addition of ethanol (750 µl) and cooling to −70°C. The solid was collected by centrifugation and washed with ethanol (1 ml). Oligonucleotide 1 (80 pmol) was added, the solution lyophilised, and the ligation was repeated.

The product (fragment I) was isolated as described above for group A, in Example 1, by electrophoresis on a 15% polyacrylamide gel in 50 mM Tris-borate pH 8.3, 1 mM EDTA. The slowest migrating band was eluted from the gel and ethanol-precipitated. An aliquot of this was examined on a denaturing gel and shown to consist of two strands of 41 and 30 bases.

Ligation of fragments 6—13 to form fragment II

Each of oligonucleotides 6 to 11 and 13 shown in Figure 6 was phosphorylated with a mixture of ATP and [γ-$^{32}$P]ATP as described for fragment I. The following aliquots were combined and ligated as described above for fragment I.

| Oligonucleotide | Amount pmol |
|---|---|
| 6 | 76 |
| 7 | 72 |
| 8 | 68 |
| 9 | 64 |
| 10 | 68 |
| 11 | 72 |
| 12 | 144 |
| 13 | 200 |

The product was isolated as described above, examined on a denaturing gel and shown to consist of two strands 45 and 54 bases as shown in Table 5.

17

TABLE 5

| | Number of bases in | | |
|---|---|---|---|
| Fragments | Oligo-nucleotides | Top strand | Bottom strand |
| I | 1—5 | 41 | 30 |
| II | 6—13 | 45 | 54 |

Synthesis of promoter

Aliquots of fragment I (3.0 pmol) and fragment II (0.6 pmol) were combined and ligated as described for fragment I except that the mixture was heated to 49°C in the ligase buffer, then cooled in a covered Dewer flask to 23°C to anneal the cohesive ends. DNA ligase was added to the mixture which was allowed to cool to 20°C by placing the covered Dewar flask in a room at 2°C. The total annealing and reaction time was 54 hours. The reaction was continued for a further 15 hours at 12°C. The DNA was precipitated from the reaction mixture with ethanol and the product was purified on a 12% polyacrylamide gel, and the slowest migrating band was eluted and ethanol precipitated. The purified product was phosphorylated with polynucleotide kinase as described in Example 1. The product was examined on a denaturing gel and shown to consist of two strands of 84 and 86 bases.

Synthesis of linker fragments

Two oligonucleotides d(CGACGATGTGT) and d(GATCACACATCGT) were synthesised as described in Example 1. These were used to join the synthetic promoter sequence to the synthetic interferon gene fragment shown in Figure 9.

It will be appreciated that the gene fragment could be synthetic, plasmid derived, or cDNA derived.

1 μg (∽0.4 pmol) EcoRI and ClaI digested pAT153 DNA was mixed with approximately 5 ng (∽0.085 pmol) phosphorylated synthetic trp promoter fragment and trated with T4 DNA ligase (BRL) under the conditions recommended by the manufacturers. After transformation of E. coli strain JA221 with 1/3 of this mixture, 10 ampicillin resistant colonies were obtained. Two of these colonies carried an approximately 85 base pair EcoRI/ClaI fragment in place of the small (25 base pair) pAT153 EcoRI/ClaI fragment. Nucleotide sequence analysis of the EcoRI/ClaI inserts in these two plasmids confirmed that they have the structure of the chemically synthesised trp promoter.

Construction of a recombinant DNA molecule comprising a Hu IFN-$\alpha_1$ gene and a synthetic trp promoter

To prepare the synthetic Hu IFN-$\alpha_1$ gene for coupling to the trp promoter, the region of the IFN gene 5' to the translation initiation codon was removed by trimming back to a Sau3A site in the region of the gene coding for amino acids 3 and 4 (Asp and Leu) of the IFN $\alpha_1$ polypeptide. To do this, a 131 base pair Sau3A/BSTEII fragment, encoding amino acids 3—45, and a 370 base pair BstEII/SalI fragment, encoding amino acid 45 to the end of the IFN gene, were isolated from plasmid pIFS1 by preparative acrylamide gel electrophoresis. These two fragments were then joined, to reconstruct the synthetic IFN gene without the region encoding the first two amino acids. This entailed ligation of the large BgIII/SalI fragment of plasmid pJDW21, to produce a plasmid designated pJDW22. (pJDW21 was constructed from pAT153 by insertion of an EcoRI/HindIII fragment of a derivative of bacteriophage λJW19, which carries the trp ΔLD102 deletion (phage stock number JW30). pJDW21 has a single BgIII site in the trp C gene it carries).

Introduction of the synthetic trp promoter fragment into pJDW22 and its fusion to an IFN gene were achieved in two stages.

Firstly, the EcoRI/ClaI trp promoter fragment from plasmid pSTP2 were inserted into EcoRI/ClaI treated pJDW22, to produce a plasmid designated pJDW30. This was possible because there is a ClaI site in pJDW22 between the EcoRI and BgIII site of pJDW22 about 1200 bp from the BgIII site. pJDW30 carries the synthetic trp promoter and the partial synthetic $\alpha_1$ IFN gene in the correct orientation to allow transcription of the IFN gene from both this promoter and the natural trp promoter of pJDW21. There are however 1200 bp between the synthetic trp promoter and the partial IFN gene and the reading frame of the trp C gene, into which the IFN gene was inserted, cannot allow a hybrid trpC/IFN polypeptide to be produced.

Fusion of the synthetic IFN gene and trp promoter was achieved by isolation of the large ClaI/BgIII fragment of pJDW30 by preparative agarose gel electrophoresis and ligation to two 5'-phosphorylated synthetic oligonucleotides as indicated in Figure 9.

These oligonucleotides are complementary to each other and enable ClaI and BgIII cohesive ends to be joined together. They regenerate the coding sequence for the first two amino acids of the synthetic Hu IFN-$\alpha_1$ (Met and Cys) and generate the same sequence between the trp attenuator ribosome binding site and the IFN gene translation initiation codon as that found in the trp ΔLD102 hybrid ribosome binding site. The plasmids derived from this construction have been designated pIFS201—pIFS230.

Construction of a wide host range plasmid comprising a Hu IFN-$\alpha_1$ gene and a synthetic *trp* promoter and transfer thereof to AS1

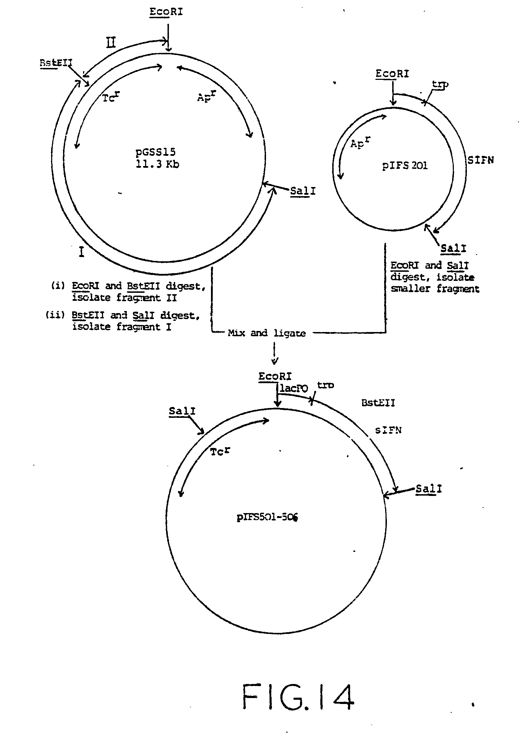

Construction of a wide host range plasmid derived from pGSS15, designated pIFS501—506, and transfer thereof to AS1 was carried out as described in Examples 3—6. Figure 14 is a diagrammatic representation of the construction of pIFS501—506.

*E. coli* strain JA221 carrying the recombinant DNA molecules pIFS201—204 and pIFS501—503 and *AS1* carrying the recombinant DNA molecules pIFS501—503 were grown to stationary phase at 37°C in Spitzizen salts medium, supplemented with 0.2: glucose; 0.05% casein hydrolysate; 20 µg/ml L-leucine; 100 µg/ml L-tryptophan; 4 µg/ml thiamine and either 33 µg/ml amplicillin or 10 µg/ml tetracycline as appropriate. 20 ml aliquots of these cultures were then centrifuged at 8000 rpm for 6 minutes, the cell pellets were then resuspended in 20 ml Spitzizen salts containing no supplements and again centrifuged. The washed cell pellets thus obtained were then resuspended in 200 ml of the above medium but containing only 2 µg/ml L-tryptophan. They were then incubated at 37° with vigorous aeration. Under these conditions the cells grew with a generation time of 45—50 minutes from an initial $A_{650}$ of 0.13—0.16 to an $A_{650}$ of 0.52—0.54, at which time they became tryptophan deficient and growth ceased. 45 minutes after growth ceased an aliquot was taken to obtain a viable cell count and the remainder of the cells were harvested and prepared for interferon assays as described in Example 1. Bioassay results are shown in Table 6.

TABLE 6

IFN Activities in E. coli JA221 and AS1 extracts

| Host | Plasmid | Promoter | IFN Units/$10^{10}$ cells |
|------|---------|----------|---------------------------|
| JA221 | pIFS201 | *trp* | $10^7$ |
| JA221 | pIFS202 | *trp* | $1.4 \times 10^7$ |
| JA221 | pIFS203 | *trp* | $5.5 \times 10^6$ |
| JA221 | pIFS204 | *trp* | $5.5 \times 10^6$ |
| JA221 | pIFS | *lac UV5* | $2 \times 10^5$ |
| JA221 | pIFS501 | *trp* | $5 \times 10^5$ |
| JA221 | pIFS502 | *trp* | $1 \times 10^6$ |
| JA221 | pIFS503 | *trp* | $7 \times 10^5$ |
| AS1 | pIFS501 | *trp* | $2.4 \times 10^6$ |
| AS1 | pIFS502 | *trp* | $3.0 \times 10^6$ |
| AS1 | pIFS503 | *trp* | $5 \times 10^6$ |

Example 8

This example describes the preparation of a Hu IFN-$\alpha_2$ gene.

Sixty eight oligodeoxynucleotides designated 1 to 68, as indicated by the arrows above and below the oligonucleotide sequence in Figure 2 were prepared as described in Example 1. HPLC purification was as described as Example 7 i.e. using 0.3 M potassium phosphate buffer (pH 6.0) in 30% or 60% formamide.

Ligation of oligonucleotides to fragments A—M

The 5'-hydroxy termini of certain of the chemically synthesised oligonucleotides were separately phosphorylated with T4 polynucloetide kinase and [γ-$^{32}$P]ATP as described in Example 1, and isolated following gel electrophoresis as described in Example 7. The phosphorylated and 5'-hydroxy oligonucleotides were assembled into thirteen groups A to M as follows.

TABLE 7

| Fragments | Oligo-nucleotides | Fragment *not* phosphorylated | Number of bases in | |
|---|---|---|---|---|
| | | | Top strand | Bottom strand |
| A | 1—5 | 1 | 44 | 32 |
| B | 6—10 | 10 | 30 | 46 |
| C | 11—15 | 11 | 45 | 30 |
| D | 16—20 | 20 | 30 | 45 |
| E | 21—25 | 21 | 45 | 30 |
| F | 26—30 | 30 | 30 | 45 |
| G | 31—35 | 31 | 46 | 30 |
| H | 36—40 | 40 | 29 | 45 |
| I | 41—45 | 41 | 46 | 30 |
| J | 46—50 | 50 | 29 | 44 |
| K | 51—55 | 51 | 45 | 31 |
| L | 56—60 | 60 | 30 | 44 |
| M | 61—68 | 68 | 58 | 55 |

The oligonucleotides required for each of the groups A to M were joined using the enzyme T4 DNA ligase, to give the double-stranded fragments A to M. These were purified by gel electrophoresis in a 15% polyacrylamide slab in the absence of urea.

Assembly of A—M into the interferon gene

Sequences (I to III) were constructed by similarly ligating the fragments A to L after phosphorylating the 5'-hydroxy termini of fragments B to L, with T4 polynucleotide kinase and ATP (containing 0.15% of [$\gamma$-$^{32}$P]ATP in groups as shown in Table 8.

TABLE 8

| Sequence | Fragments | Number of bases in | |
|---|---|---|---|
| | | Top strand | Bottom strand |
| I | ABCD | 149 | 153 |
| II | EFGH | 150 | 150 |
| III | IJKL | 150 | 149 |

The sequences I to III were purified by gel electrophoresis in a 10% polyacrylamide slab and ligated with fragment M to give the Hu IFN-$\alpha_2$ gene (Figure 2), which was purified by gel electrophoresis in a 6% polyacrylamide slab.

Example 9

This example describes the production of interferons by E. coli cells which comprise a copy interferon gene prepared from the mRNA of Namalva cells.

Induction of interferon in Namalwa cells for mRNA extraction

Namalwa cells were grown as suspension cultures in spinner flasks using RPMI 1640 supplemented with 10% foetal calf serum and 1 mM L-glutamine. For induction, the cells ($2 \times 10^6$ ml) in RPMI 1640 supplemented with 2% foetal calf serum and L-glutamine (1 mM) were treated with butyric acid (1 mM) for 48 hour, spundown, washed once with RPMI 1640 supplemented with 5% newborn calf serum and resuspended in the same medium at $3 \times 10^7$ cells/ml. Sendai virus (strain AE101) in PBS was then added at a concentration of 3000 HAU/ml and the cell suspension incubated at 37°C for 1 hour. Nine volumes of RPMI 1640 supplemented with 5% newborn calf serum and L-glutamine (1 mM) were then added and the cultures were incubated at 37°C for nine hours.

The induced cells were harvested, washed twice with ice cold PBS and resuspended in 2 volumes of 50 mM HEPES (pH 7.6), 240 ml KCl, 10 mM $MgSO_4$, 3 mM DTT, 250 mM sucrose, 2% NP-40 and 10 mM vanadyl-ribonucleoside complex (BRL). The suspended cells were lysed by vortexing in this buffer for 1 minute and the lysate centrifuged for 15 minutes at 23,000 g. The resulting 23,000 g supernate was made 10 mM with vanadyl-ribonucleoside complex and 20 mM with streptomycin sulphate (from a fresh 10× stock) to precipitate polysomes. The preparation was left at 0°C for 3 hours. At the end of this time, the precipitated polysomes were centrifuged for 15 minutes at 5000 g. The pellet was then resuspended in 10 mM Tris-HCl (pH 7.6), 0.4M NaCl, 1% SDS and passed twice through 1 ml oligo dT-cellulose (PL Biochemicals) columns. The poly $A^+$ polysomal RNA was eluted from the column, precipitated with ethanol, translated in reticulocyte lysates (Amersham), and the translation reaction was titered for interferon activity. 0.2 μg of mRNA in a 20 μl reaction yielded ∞80 units of interferon. The interferon activity obtained in this way was totally neutralised by antiserum prepared against Namalwa interferon.

The total poly $A^+$ RNA was further fracionated on a 5—30% sucrose gradient prepared in 50 mM Tris (pH 7.6), 3 M NaCl, 10 mM EDTA, 0.5 SDS. The gradient was centrifuged in an SW27 rotor at 26,500 rpm for 17 hours. The gradient was collected from the bottom and all fractions sedimenting faster than 18S and slower than 9S were pooled. The fractions in between were ethanol precipitated and individually translated in reticulocyte lysates. The translation reactions were titrated and a peak of interferon mRNA was found at approximately 12—16S. This mRNA was pooled and ethanol precipitated to be used for cDNA production and cloning.

Synthesis of double-stranded cDNA from sucrose gradient enriched mRNA

Single-stranded cDNA was prepared in a 50 μl reaction containing 5 μg of 12—16S fraction mRNA, 50 mM Tris-HCl (pH 8.3), 50 mM NaCl, 8 mM $MgCl_2$, 10 mM DTT, 5 μCi $^3HdCTP$ (21 ci/mmol, Amersham International), 1 mM dATP, 1 mM dGTP, 1 mM dCTP, 1 mM TTP, 5 μg oligo $dT_{12-18}$ (PL Biochemicals) and 17 units AMV reverse transcriptase (batch G380). The reaction mixture was incubated at 42°C for 60 minutes, then diluted with 50 μl 5 mM Tris-HCl (pH 8.3), 5 mM DTT; a further 17 units of reverse transcriptase was then added and a further 30 minutes incubation at 47° was given to try to ensure complete copying of any potentially secondary structures in the mRNA (Dr. A. Dunn, personal communication). The reaction was terminated by addition of 2 μl 0.4 M EDTA, 2 μl 10% SDS and phenol extraction. cDNA and residual RNA were then separated from unincorporated nucleotides by gel-filtration on Sephadex G50 Fine in 10 mM Tris-HCl (pH 7.5); 1 mM EDTA. Excluded fractions were then made 0.2 M in NaOH and incubated at 67° for 10 minutes before neutralisation and ethanol precipitation overnight at −20°.

The second strand cDNA synthesis was performed essentially as described by Kay *et al*, Nucleic Acids Research, *8* 2691 (1980) using 1.1 μg single-stranded cDNA as a template and $α^{32}P$ dCTP to follow the reaction.

0.45 μg double-stranded cDNA was treated with approximately 300 units S1 nuclease (BRL) for 10 minutes at 35° in 250 mM NaCl; 30 mM sodium acetate (pH 4.5), 4.5 mM $ZnCl_2$. The reaction was terminated by addition of EDTA and SDS and phenol extraction, as above; cDNA was separated from nucleotides by Sephadex G50 Fine gel filtration. After ethanol precipitation, the double-stranded cDNA was then fractionated by electrophoresis on a 5% polyacrylamide gel in TBE buffer (Maniatis *et al*., Biochemistry, *14*, 3787, (1975), a fraction greater than 600 bp in size was recovered from the gel by electroelution, DE52 chromatography and ethanol precipitation.

44 ng of size fractionated cDNA was tailed with approximately 25 dCMP residues/3' terminus with terminal deoxynucleotidyl transferase (TdT; PL) (Roy Choudhury *et al*. Nucleic Acids Research *3* 863 (1976)). It was then recovered by phenol extraction, gel filtration and ethanol precipitation.

Construction of recombinant plasmids

The double-stranded, size fractionated, dCMP tailed cDNA, prepared as described above, was mixed with an approximately equimolar amount (150 ng) of pAT153 DNA, which had previously been *Pstl* cleaved and tailed with approximately 25 dGMP residues/3' terminus, in 200 μl 100 mM NaCl, 10 mM Tris-HCl (pH 7.5), 0.2 mM EDTA. The mixture was incubated for 5 minutes at 65°, 2 hours at 44° and then allowed to cool slowly (in a switched-off water bath) to room temperature overnight, to allow annealing of the oligo dC and dG tails to occur. Itw as then stored on ice for several days during which time aliquots were used to transform *E. coli* strain MRV8 cells, which had been made competent by treatment with 0.1 M $CaCl_2$ supplemented with N-acetylglucosamine (0.4 mg/ml) and 2,6-diaminopimelic acid (0.1 mg/ml). 3000 tetracycline resistant ($Tc^r$) transformants were obtained. Of these, 2450 (81.7%) apparently contain recombinant DNA as shown by their sensitivity to penicillin.

Analysis of the cDNA clones

(i) Colony hybridisation

The transformed bacteria were plated onto nitrocellulose filters (Schliecher and Schnell, 82 mm, BA85) placed on L-broth agar plates containing 10 µg/ml tetracycline. The filters were hybridised, as described by Grunstein and Hogness, Proceedings of the National Academy of Sciences, USA, *72*, 3961, 1975, with a $^{32}$P-labelled nick translated probe (Rigby *et al.*, Journal of Molecular Biology, *113*, 237, 1977) generated from a chemically synthesised DNA fragment based on part of the sequence (corresponding to amino acids 78—97) of the Hu IFN-$\alpha_1$ gene shown in Figure 1. Groups of 10 filters were incubated with approximately $10^6$ Cherenkov cpm in 5× Denhardts at either 37°C or 50°C, overnight. The filters were then washed four times for 15 minutes each in 5× Denhardts containing 1% SDS at the same temperature as the hybridisation had been performed and finally for 1 hour in 3×SSC+1% SDS. The filters were dried and exposed to Kodak X-omatic H film and a Kodak intensifying screen.

From this hybridisation experiment four clones, hereinafter referred to for convenience as pcIF24.40, pcIF24.47, pcIF29.43 and pcIF41.13, were detected which carry cDNA species closely related to the leucocyte IFN gene family.

(ii) Interferon bioassays

Bioassays were performed on extracts produced from pooled cultures of 10 individual colonies. Where the pools were positive, individual colonies were then investigated. In the case of pooled cultures, 10 ml cultures of individual clones were grown to early stationary phase (approx. $10^9$ cells/ml) and pools of 10×10 ml cultures prepared. For the assay of individual colonies, 100 ml cultures were grown. The cells were harvested and resuspended in 0.01 volumes of 50 mM Tris-HCl (pH 8.0), 50 mM EDTA and 15% sucrose, lysozyme was added to a final concentration of 2.5 mg/ml and the suspension kept on ice for 30 minutes. The samples were then frozen at −20°C overnight, thawed at room temperature and sonicated for 15 seconds. Extracts were kept at −20°C until used for bioassay. Serial two-fold dilutions of the samples were made in 96 well microtitre plates; T98G cells (a human neuroblastoma line) were added to each well (40,000 cells per well) and the cultures incubated overnight at 37°C. The cultures were then challenged with EMC virus (0.6 PFu/cell) and scored for cytopathic effect 24—30 hours later. The titre of the sample (expressed as units/0.15 ml) is defined as the reciprocal of the maximum dilution reducing the cytopathic effect by 50% or more. In this assay, the NIH human reference standard G-023-901-527 (20,000 µ/ml) diluted 1 in 50 and hence containing by definition 400 Iu/ml gave a titre of 120 units/0.15 ml.

300 extracts, prepared from pools of 10 clones, were assayed for antiviral activity. 35 extracts were scored as having such activity. Of these 35 pools, 30 were re-tested in the presence of antiserum to Namaliva IFN and in 11 cases the activity was neutralised.

Extracts of the individual members of 5 pools were assayed for antiviral activity and seven were found to be positive. A typical result for one clone pcIF56.2 is shown in Table 9.

TABLE 9

| Clone | Interferon titre (units/1.5×10⁶ cells)[a] | | |
| --- | --- | --- | --- |
| | Experiment 1 | Experiment 2 | Experiment 3 |
| 56.2 | 600 | 1200 | 1200 |
| 56.2+antiserum | b | 300 | 300 |

[a]: Assayed on T98G cells challenged with EMC virus.
[b]: Not determined.

Restriction endonuclease maps and DNA sequence analysis of the cDNA clones

cDNA clones 24.40, 24.47, 29.43, 41.13 and 56.2 have been mapped using the restriction enzymes *Pvu*II, *Bgl*II and *Eco*RI. It has previously been shown that different IFN genes can be grouped into eight classes on the basis of their restriction maps with these enzymes (Goeddel et al, 1981, Nature, *290*, 20—26). Figure 15 shows the restriction maps obtained for the 4 clones (pcIF24.40, pcIF24.47, pcIF29.43 and pcIF41.13) identified by hybridisation and for 1 clone (pcIF56.2) identified in the bioassay screen.

Clone pcIF24.40 appears to be very similar in its restriction map with that described for leucocyte IFNE although it also contains an additional *Pvu*II site in a position which would correspond to that present in the sequences of leucocyte IFN A, D and H (Goeddel et al. etc.). This *Pvu*II site is in the sequence for the signal peptide of the interferon coded for by these clones. The leucocyte IFNE cDNA sequence previously described did not extend this far.

Clone pcIF56.2 does not contain any restriction enzyme sites for the three enzymes tested and on further analysis it was shown that there were also no restriction sites for *Hpa*II, *Pst*I, or *Taq*I. The only restriction site we have been able to assign to this cDNA to date is a single AluI site (Figure 15). This and the fact that this clone does not hybridise to the synthetic oligonucleotide probe is indecative that this cDNA is not related to any of the interferon sequences previously described. Figure 16 shows a partial DNA sequence of this clone. It can be observed that there is an extensive open reading frame but there is no DNA or amino acid sequence in common with any previously described IFN.

Characterisation of the products expressed by the cDNA clones

Clone pcIF56.2 was identified by the direct bioassay screening approach. Because it does not have any sequence homology with other known IFN's it was important to demonstrate properties of this clone which are compatible with it expressing a product which has IFN activity. These properties can be summarised as follows:—

(i) The cloned DNA binds IFN mRNA from Namalwa cells in a filter binding assay (Nagata et al, 1980, Nature, *284*, 316—320).

(ii) The product expressed by this clone exhibits antiviral activity, stable at pH2 which
—is neutralised by anti-Namalwa IFN serum
—is retained on an antibody affinity column (anti-leucocyte IFN serum)
—is partially retained on blue sepharose and poly U columns.

(iii) The product stimulates the activity of the enzyme 2',5'-oligoabenylate synthetase.

This evidence suggests that pcIF56.2 produces a novel polypeptide with biological properties expected for IFN. The cDNA clones were constructed by annealing deoxy C tailed cDNA to pAT153 DNA which had been cleaved in the β-lactamase gene by *Pst*I digestion and tailed with deoxy G residues. *Pst*I sites are regenerated at both ends of the insert as a result of such recircularisation (Otsuka A, 1981, Gene, *13*, 339). Plasmid DNA was isolated from clone pcIF24—40 and the cDNA insert was recovered by digestion with *Pst*I and was mixed with plasmid pGSS15 DNA which had been linearised by digestion with *Pst*I. The mixture was ligated and transferred into *E. coli* PA340.22. Transformants containing the cDNA insert were isolated and extracts of these bacteria were assayed for IFN activity. A number of these extracts, particularly from clone pGSS15/24.40:A11, have consistently been shown to have an antiviral activity stable at pH2. Furthermore this antiviral activity is neutralised both by the anti-Namalwa IFN serum and two independent anti-leucocyte IFN sera, but not by two independent anti-fibroblast IFN sera. This evidence would suggest that clone pGSS15/24.40:A11 produces a product with the characteristics expected for Hu IFN-α.

The expression of cDNA clones in M. methylotrophus (AS1)

pGSS15/24.40 A11 was transferred from *E. coli* to AS1 as described in Examples 3—6. The production of interferon in both organisms was confirmed by bioassays of bacterial extracts as described previously. These results are shown in Table 10.

TABLE 10

| IFN cDNA in | Antiviral titre (units/0.15 ml) |
|---|---|
| E. coli | 1200 |
| M. methylotrophus | 495 |

## Claims

1. A process for the preparation of genetic material coding for a compound having interferon activity per se or capable of being modified in vivo or in vitro to provide a compound having interferon activity characterised in that the process comprises at least the steps of

(A) chemically synthesising fragments of the genetic material each of which fragments contains four to six inclusive oligonucleotides which are alignable through hydrogen bonding to the complementary strand;

(B) preparing sequences by joining two to three appropriate fragments which are substantially free of intra- and inter-complementarity; and

(C) joining the said sequences in the appropriate order; such that the said genetic material is prepared with the proviso that sites recognisable by restriction enzymes used in the latter stages of the process are avoided.

2. A process as claimed in claim 1 wherein the oligonucleotides used in Step A are chosen from those hereinafter designated 1a to 67a:

| Oligonucleotide designation | Bases | Fragments | Sequences |
|---|---|---|---|
| 1a | GATCCATGTGTGAT | Aa | Ia |
| 2a | GTACACACTAGACGGCCT | | |
| 3a | CTGCCGGAGACCCAT | | |
| 4a | CTGGGTATCGGACCT | | |
| 5a | AGCCTGGATAACCGT | | |
| 6a | ATTGGCAGCATGGGA | Ba | |
| 7a | CGTACCCTGATGCTG | | |
| 8a | CTACGACGACCGTGT | | |
| 9a | CTGGCACAAATGAGC | | |
| 10a | TTACTCGGCGTAGAGA | | |
| 11a | CGCATCTCTCCGTCC | | |
| 12a | GGCAGGAGGACAGA | Ca | |
| 13a | TCCTGTCTAATGGAC | | |
| 14a | TTACCTGTCTGTACT | | |
| 15a | AGACATGACTTTGGCT | | |
| 16a | GAAACCGAAAGGGGT | | |
| 17a | TTCCCCAAGAGGAGT | Da | IIa |
| 18a | TCTCCTCAAGCTACCA | | |
| 19a | TCGATGGTAACCAGT | | |
| 20a | TTGGTCAAGGTCTTC | | |
| 21a | TCCAGAAGGCACCGG | | |
| 22a | CGTGGCCGATAGTCG | | |
| 23a | CTATCAGCGTACTCC | | |
| 24a | CATGAGGTGCTTGACT | Ea | |
| 25a | ACGAACTGATCCAAC | | |
| 26a | AGGTTGTTTAGAAG | | |
| 27a | AAATCTTCAACCTCT | | |
| 28a | TTGGAGAAATGGTGA | | |
| 29a | TTACCACTAAAGACT | | |
| 30a | TTTCTGAGTAGACGA | Fa | IIIa |
| 31a | CATCTGCTGCTTGGG | | |
| 32a | CGAACCCTGCTCCTA | | |
| 33a | ACGAGGATCTGCTAG | | |
| 34a | GACGATCTGTTTAA | | |
| 35a | ACAAATTCTGCACCGA | | |
| 36a | GACGTGGCTTGAGAT | | |
| 37a | ACTCTACCAGCAAC | | |
| 38a | GGTCGTTGACTTGCT | Ga | |
| 39a | TGAACGACCTGGAAG | | |
| 40a | GGACCTTCGGACACA | | |
| 41a | CCTGTGTAATGCAAG | | |
| 42a | TTACGTTCTTCTCTC | | |
| 43a | AAGAGAGAGTGGGCG | | |
| 44a | TCACCCGCTTTGAGG | Ha | |
| 45a | AAACTCCGCTGATGAAT | | |
| 46a | CGACTACTTACGCCTGA | | |
| 47a | GCGGACTCCATCCTG | | |
| 48a | GGTAGGACCGACAAT | | |
| 49a | GCTGTTAAAAAGTAT | | |
| 50a | TTTTCATAAAGGCAT | Ia | IVa |
| 51a | TTCCGTAGAATCACC | | |
| 52a | CTTAGTGGGACATAGA | | |
| 53a | CTGTATCTGACTGAG | | |
| 54a | CTGACTCTTTTTCATA | | |
| 55a | AAAAAGTATAGCCCG | | |
| 56a | TCGGGCACGCGAA | | |
| 57a | TGCGCTTGGGAGGTT | Ja | |
| 58a | CCCTCCAACAGGCAC | | |
| 59a | GTCCGTGCTGAGATC | | |
| 60a | GACTCTAGTACGCAA | | |
| 61a | ATGCGTTCCCTGTCTT | | |

24

| Oligonucleotide designation | Bases | Fragments | Sequences |
|---|---|---|---|
| 62a | GGGACAGAAATAGTTGG | Ka | |
| 63a | TATCAACCAATCTTC | | |
| 64a | TTAGAAGTTCTTGCAAA | | |
| 65a | AAGAACGTTTAAGGCGC | | |
| 66a | TTCCGCGTTCCTTATTCAGCT | | |
| 67a | AAGGAATAAG | | |

3. A process as claimed in claim 1 wherein the oligonucleotides used in step A are chosen from those hereinafter designated 1b—68b:

| Oligonucleotide designation | Bases | Fragments | Sequences |
|---|---|---|---|
| 1b | GATCCATGTGTGAT | Ab | Ib |
| 2b | GTACACACTAGACGGC | | |
| 3b | CTGCCGCAAACTCAT | | |
| 4b | GTTTGAGTATCGGACC | | |
| 5b | AGCCTGGGTAGCCGT | | |
| 6b | CATCGGCAGCGTGGGA | Bb | |
| 7b | CGCACCCTGATGCTG | | |
| 8b | CTACGACGACCGGGT | | |
| 9b | CTGGCCCAAATGCGC | | |
| 10b | TTACGCGGCATAGAG | | |
| 11b | CGTATCTCCCTGTTC | Cb | |
| 12b | GGACAAGAGGACAGA | | |
| 13b | TCCTGTCTGAAAGAC | | |
| 14b | CTTTCTGGCGGTACT | | |
| 15b | CGCCATGACTTTGGC | | |
| 16b | GAAACCGAAGGGCGT | Db | |
| 17b | TTCCCGCAGGAAGAG | | |
| 18b | CCTTCTCAAGCCATT | | |
| 19b | TTCGGTAACCAGTTC | | |
| 20b | GGTCAAGGTTTTCCG | | |
| 21b | CAAAAGGCAGAAACT | Eb | IIb |
| 22b | TCTTTGATAGGGCCA | | |
| 23b | ATCCCGGTACTGCAC | | |
| 24b | TGACGTGCTTTACTA | | |
| 25b | GAAATGATTCAACAG | | |
| 26b | AGTTGTCTAAAAATT | Fb | |
| 27b | ATTTTTAACCTGTTC | | |
| 28b | GGACAAGTCGTGATT | | |
| 29b | AGCACTAAAGACTCC | | |
| 30b | TCTGAGGAGACGACG | | |
| 31b | TCTGCTGCATGGGAC | Gb | |
| 32b | TACCCTGCTTTGAGA | | |
| 33b | GAAACTCCTGGAC | | |
| 34b | GGACCTGTTTAAGAT | | |
| 35b | AAATTCTACACCGAAC | | |
| 36b | GTGGCTTGACATGGT | Hb | |
| 37b | TGTACCAGCAACTG | | |
| 38b | CGTTGACTTGCTGGA | | |
| 39b | AACGACCTGGAAGCC | | |
| 40b | CCTTCGGACGCAGTA | | |
| 41b | TGCGTCATCCAGGGT | Ib | IIIb |
| 42b | GGTCCCACAACCGCA | | |
| 43b | GTTGGCGTAACCGAA | | |
| 44b | TTGGCTTTGAGGCGA | | |
| 45b | ACTCCGCTGATGAAAG | | |
| 46b | CTACTTTCTTCTGAG | Jb | |
| 47b | AAGACTCCATCCTG | | |
| 48b | GTAGGACCGACAAGC | | |
| 49b | GCTGTTCGCAAATAC | | |
| 50b | GTTTATGAAGGTCG | | |

25

| Oligonucleotide designation | Bases | Fragments | Sequences |
|---|---|---|---|
| 51b | TTCCAGCGTATCACC | Kb | |
| 52b | CATAGTGGGACATGGA | | |
| 53b | CTGTACCTGAAAGAG | | |
| 54b | CTTTCTCTTCTTTAT | | |
| 55b | AAGAAATACAGCCCG | | |
| 56b | GTCGGGCACGCGAAC | Lb | |
| 57b | TGCGCTTGGGAAGTT | | |
| 58b | CCTTCAACATGCGC | | |
| 59b | GTACGCGCTGAAATC | | |
| 60b | GACTTTAGTACGCAA | | |
| 61b | ATGCGTTCCTTCAGC | Mb | |
| 62b | GGAAGTCGGACAGGT | | |
| 63b | CTGTCCACTAACCTG | | |
| 64b | GATTGGACGTTCTTA | | |
| 65b | CAAGAATCTCTGCGT | | |
| 66b | GAGACGCATCGTTTC | | |
| 67b | AGCAAAGAATAAG | | |
| 68b | TTATTCAGCT | | |

4. A process as claimed in claim 1 wherein the fragments used in step B are selected from those designated Aa to Ka in claim 2.

5. A process as claimed in claim 1 wherein the fragments used in step B are those selected from those designated Ab to Mb in claim 3.

6. A process as claimed in claim 1 wherein the sequences used in step C are selected from those designated Ia to IVa in claim 2.

7. A process as claimed in claim 1 wherein the sequences used in step C are selected from those designated Ib to IIIB in claim 3.

8. A method for the preparation of a recombinant DNA molecule which molecule comprises a suitable vector and a gene which encodes a compound having interferon activity per se or capable of being modified in vivo or in vitro to provide a compound having interferon activity characterised in that (i) the gene is synthesised by a process as claimed in any one of claims 1—7 and (ii) that the gene is joined to the vector such that it is under the transcriptional control of a suitable promoter and on transcription yields an mRNA molecule in which the translation initiation codon of the RNA sequence encoding the said compound is fused in the region of a ribosome-binding site such that production of a hybrid polypeptide does not occur.

9. A method as claimed in claim 6 or 7 wherein the vector is a composite vector derived from R300B.

10. A method as claimed in claim 6 or 7 wherein the promoter is a *lac* promoter.

11. A method as claimed in claim 6 or 7 wherein the promoter is a *trp* promoter.

12. A method as claimed in claim 11 wherein the *trp* promoter is a synthetic analogue of a trp ΔLD102 promoter.

13. A process for the preparation of a genetically modified microorganism capable of expressing as a metabolite a compound having interferon activity per se or capable of being modified in vivo or in vitro to provide a compound having interferon activity and comprising genetic material coding for said metabolite characterised in that the process comprises the steps of (i) a chemically synthesising the genetic material by a process as claimed in claims 1—7; (ii) synthesising a recombinant DNA molecule by a method as claimed in claims 8—12; and (iii) infecting the microorganism with the recombinant DNA molecule.

14. A process as claimed in claim 13 wherein the microorganism is an autotrophic or methylotrophic bacterium selected from Methylophilus methylotrophus, pseudomonas methylonica, pseudomonas utilis, pseudomonas inaudita, methylomonas clara or Alcaligenes eutropha.

**Patentansprüche**

1. Verfahren zur Herstellung eines genetischen Materials, das für eine Verbindung codiert, das per se Interferonaktivität aufweist oder in der Lage ist, in vivo oder in vitro so modifiziert zu werden, daß eine Verbindung mit Interferonaktivität erhalten wird, dadurch gekennzeichnet, daß das Verfahren wenigstens die Stufen umfaßt

(A) auf chemischem Wege Synthetisieren von Fragmenten des genetischen Materials, wobei jedes dieser Fragmente vier bis einschließlich sechs Oligonukleotide enthält, die durch Wasserstoff-Brückenbindung am Komplementärstrang aufreihbar sind;

(B) Herstellen von Sequenzen durch Verbinden von zwei bis drei geeigneten Fragmenten, die im wesentlichen frei von Intra- und Inter-Komplementärität sind; und

(C) Verbinden dieser Sequenzen in der richtigen Reihenfolge, so daß das genetische Material hergestellt wird mit der Maßgabe, daß Stellen, die von in späteren Stufen des Verfahrens verwendeten Restriktionsenzymen erkannt werden können, vermieden werden.

2. Verfahren nach Anspruch 1, bei dem die in Stufe A verwendeten Oligonukleotide aus denen ausgewählt werden, die nachfolgend mit 1a bis 67a bezeichnet werden:

| Oligonukleotid Bezeichnung | Basen | Fragmente | Sequenzen |
|---|---|---|---|
| 1a | GATCCATGTGTGAT | Aa | Ia |
| 2a | GTACACACTAGACGGCCT | | |
| 3a | CTGCCGGAGACCCAT | | |
| 4a | CTGGGTATCGGACCT | | |
| 5a | AGCCTGGATAACCGT | | |
| 6a | ATTGGCAGCATGGGA | Ba | |
| 7a | CGTACCCTGATGCTG | | |
| 8a | CTACGACGACCGTGT | | |
| 9a | CTGGCACAAATGAGC | | |
| 10a | TTACTCGGCGTAGAGA | | |
| 11a | CGCATCTCTCCGTCC | | |
| 12a | GGCAGGAGGACAGA | Ca | |
| 13a | TCCTGTCTAATGGAC | | |
| 14a | TTACCTGTCTGTACT | | |
| 15a | AGACATGACTTTGGCT | | |
| 16a | GAAACCGAAAGGGGT | | |
| 17a | TTCCCCAAGAGGAGT | | |
| 18a | TCTCCTCAAGCTACCA | Da | IIa |
| 19a | TCGATGGTAACCAGT | | |
| 20a | TTGGTCAAGGTCTTC | | |
| 21a | TCCAGAAGGCACCGG | | |
| 22a | CGTGGCCGATAGTCG | | |
| 23a | CTATCAGCGTACTCC | | |
| 24a | CATGAGGTGCTTGACT | Ea | |
| 25a | ACGAACTGATCCAAC | | |
| 26a | AGGTTGTTTAGAAG | | |
| 27a | AAATCTTCAACCTCT | | |
| 28a | TTGGAGAAATGGTGA | | |
| 29a | TTACCACTAAAGACT | | |
| 30a | TTCTGAGTAGACGA | Fa | IIIa |
| 31a | CATCTGCTGCTTGGG | | |
| 32a | CGAACCCTGCTCCTA | | |
| 33a | ACGAGGATCTGCTAG | | |
| 34a | GACGATCTGTTTAA | | |
| 35a | ACAAATTCTGCACCGA | | |
| 36a | GACGTGGCTTGAGAT | | |
| 37a | ACTCTACCAGCAAC | | |
| 38a | GGTCGTTGACTTGCT | Ga | |
| 39a | TGAACGACCTGGAAG | | |
| 40a | GGACCTTCGGACACA | | |
| 41a | CCTGTGTAATGCAAG | | |
| 42a | TTACGTTCTTCTCTC | | |
| 43a | AAGAGAGAGTGGGCG | | |
| 44a | TCACCCGCTTTGAGG | Ha | |
| 45a | AAACTCCGCTGATGAAT | | |
| 46a | CGACTACTTACGCCTGA | | |
| 47a | GCGGACTCCATCCTG | | |
| 48a | GGTAGGACCGACAAT | | |
| 49a | GCTGTTAAAAAGTAT | | |
| 50a | TTTTCATAAAGGCAT | Ia | IVa |
| 51a | TTCCGTAGAATCACC | | |
| 52a | CTTAGTGGGACATAGA | | |
| 53a | CTGTATCTGACTGAG | | |
| 54a | CTGACTCTTTTTCATA | | |
| 55a | AAAAAGTATAGCCCG | | |
| 56a | TCGGGCACGCGAA | | |

| Oligonukleotid Bezeichnung | Basen | Fragmente | Sequenzen |
|---|---|---|---|
| 57a | TGCGCTTGGGAGGTT | Ja | |
| 58a | CCCTCCAACAGGCAC | | |
| 59a | GTCCGTGCTGAGATC | | |
| 60a | GACTCTAGTACGCAA | | |
| 61a | ATGCGTTCCCTGTCTT | | |
| 62a | GGGACAGAAATAGTTGG | Ka | |
| 63a | TATCAACCAATCTTC | | |
| 64a | TTAGAAGTTCTTGCAAA | | |
| 65a | AAGAACGTTTAAGGCGC | | |
| 66a | TTCCGCGTTCCTTATTCAGCT | | |
| 67a | AAGGAATAAG | | |

3. Verfahren nach Anspruch 1, bei dem die in Stufe A verwendeten Oligonukleotide aus denen ausgewählt sind, die nachfolgend mit 1b bis 68b bezeichnet sind:

| Oligonukleotid Bezeichnung | Basen | Fragmente | Sequenz |
|---|---|---|---|
| 1b | GATCCATGTGTGAT | Ab | Ib |
| 2b | GTACACACTAGACGGC | | |
| 3b | CTGCCGCAAACTCAT | | |
| 4b | GTTTGAGTATCGGACC | | |
| 5b | AGCCTGGGTAGCCGT | | |
| 6b | CATCGGCAGCGTGGGA | Bb | |
| 7b | CGCACCCTGATGCTG | | |
| 8b | CTACGACGACCGGGT | | |
| 9b | CTGGCCCAAATGCGC | | |
| 10b | TTACGCGGCATAGAG | | |
| 11b | CGTATCTCCCTGTTC | Cb | |
| 12b | GGACAAGAGGACAGA | | |
| 13b | TCCTGTCTGAAAGAC | | |
| 14b | CTTTCTGGCGGTACT | | |
| 15b | CGCCATGACTTTGGC | | |
| 16b | GAAACCGAAGGGCGT | Db | |
| 17b | TTCCCGCAGGAAGAG | | |
| 18b | CCTTCTCAAGCCATT | | |
| 19b | TTCGGTAACCAGTTC | | |
| 20b | GGTCAAGGTTTTCCG | | |
| 21b | CAAAAGGCAGAAACT | Eb | IIb |
| 22b | TCTTTGATAGGGCCA | | |
| 23b | ATCCCGGTACTGCAC | | |
| 24b | TGACGTGCTTTACTA | | |
| 25b | GAAATGATTCAACAG | | |
| 26b | AGTTGTCTAAAAATT | Fb | |
| 27b | ATTTTTAACCTGTTC | | |
| 28b | GGACAAGTCGTGATT | | |
| 29b | AGCACTAAAGACTCC | | |
| 30b | TCTGAGGAGACGACG | | |
| 31b | TCTGCTGCATGGGAC | Gb | |
| 32b | TACCCTGCTTTGAGA | | |
| 33b | GAAACTCTCCTGGAC | | |
| 34b | GGACCTGTTTAAGAT | | |
| 35b | AAATTCTACACCGAAC | | |
| 36b | GTGGCTTGACATGGT | Hb | |
| 37b | TGTACCAGCAACTG | | |
| 38b | CGTTGACTTGCTGGA | | |
| 39b | AACGACCTGGAAGCC | | |
| 40b | CCTTCGGACGCAGTA | | |

EP 0 062 971 B1

| Oligonukleotid Bezeichnung | Basen | Fragmente | Sequenz |
|---|---|---|---|
| 41b | TGCGTCATCCAGGGT | Ib | IIIb |
| 42b | GGTCCCACAACCGCA | | |
| 43b | GTTGGCGTAACCGAA | | |
| 44b | TTGGCTTTGAGGCGA | | |
| 45b | ACTCCGCTGATGAAAG | | |
| 46b | CTACTTTCTTCTGAG | Jb | |
| 47b | AAGACTCCATCCTG | | |
| 48b | GTAGGACCGACAAGC | | |
| 49b | GCTGTTCGCAAATAC | | |
| 50b | GTTTATGAAGGTCG | | |
| 51b | TTCCAGCGTATCACC | Kb | |
| 52b | CATAGTGGGACATGGA | | |
| 53b | CTGTACCTGAAAGAG | | |
| 54b | CTTTCTCTTCTTTAT | | |
| 55b | AAGAAATACAGCCCG | | |
| 56b | GTCGGGCACGCGAAC | Lb | |
| 57b | TGCGCTTGGGAAGTT | | |
| 58b | CCTTCAACATGCGC | | |
| 59b | GTACGCGCTGAAATC | | |
| 60b | GACTTTAGTACGCAA | | |
| 61b | ATGCGTTCCTTCAGC | Mb | |
| 62b | GGAAGTCGGACAGGT | | |
| 63b | CTGTCCACTAACCTG | | |
| 64b | GATTGGACGTTCTTA | | |
| 65b | CAAGAATCTCTGCGT | | |
| 66b | GAGACGCATCGTTTC | | |
| 67b | AGCAAAGAATAAG | | |
| 68b | TTATTCAGCT | | |

4. Verfahren nach Anspruch 1, bei dem die in Stufe B verwendeten Fragmente aus denen ausgewählt sind, die in Anspruch 2 mit Aa bis Ka bezeichnet sind.

5. Verfahren nach Anspruch 1, bei dem die in Stufe B verwendeten Fragmente solche sind, die aus denen ausgewählt sind, die in Anspruch 3 mit Ab bis Mb bezeichnet sind.

6. Verfahren nach Anspruch 1, bei dem die in Stufe C verwendeten Sequenzen aus denen ausgewählt sind, die in Anspruch 2 mit Ia bis IVa bezeichnet sind.

7. Verfahren nach Anspruch 1, bei dem die in Stufe C verwendeten Sequenzen aus denen ausgewählt sind, die in Anspruch 3 mit Ib bis IIIb bezeichnet sind.

8. Verfahren zur Herstellung eines rekombinanten DNA-Moleküls, wobei dieses Molekül einen geeigneten Vektor und ein Gen umfaßt, das für eine Verbindung kodiert, die per se Interferonaktivität aufweist oder in der Lage ist, in vivo oder in vitro so modifiziert zu werden, daß eine Verbindung mit Interferonaktivität geschaffen wird, dadurch gekennzeichnet, daß

(i) das Gen nach einem Verfahren synthetisiert wurde, wie es in einem der Ansprüche 1 bis 7 beansprucht wird, und daß

(ii) das Gen so mit dem Vektor verknüpft ist, daß es unter der Transkriptionskontrolle eines geeigneten Promoters steht und bei der Transkription ein mRNA-Molekül liefert, in dem das Startkodon für die Translation der RNA Sequenz, die für die genannte Verbindung kodiert, in den Bereich einer ribosomalen Bindungsstelle eingebunden ist, so daß es nicht zur Erzeugung eines Hybridpolypeptids kommt.

9. Verfahren nach Anspruch 6 oder 7, bei dem der Vektor ein von R300B abgeleiteter Verbundvektor ist.

10. Verfahren nach Anspruch 6 oder 7, bei dem der Promoter ein *lac*-Promotor ist.

11. Verfahren nach Anspruch 6 oder 7, bei dem der Promoter ein *trp*-Promotor ist.

12. Verfahren nach Anspruch 11, bei dem der *trp*-Promotor ein synthetisches Analogon eines trp ΔLD102-Promotors ist.

13. Verfahren zur Herstellung eines genetisch modifizierten Mikroorganismus, der in der Lage ist, als einen Metaboliten eine Verbindung zu exprimieren, die per se Interferonaktivität aufweist oder in der Lage ist, in vivo oder in vitro modifiziert zu werden, so daß eine Verbindung mit Interferonaktivität erhalten wird, und der genetisches Material enthält, das für den genannten Metaboliten kodiert, dadurch gekennzeichnet, daß das Verfahren die Stufen

(i) der Synthese auf chemischem Wege des genetischen Materials nach einem Verfahren nach den Ansprüchen 1 bis 7;

(ii) der Synthese eines rekombinanten DNA-Moleküls nach einem Verfahren nach den Ansprüchen 8 bis 12; und

(iii) des Infizierens des Mikroorganismus mit dem rekombinanten DNA-Molekül umfaßt.

29

14. Verfahren nach Anspruch 13, bei dem der Mikroorganismus ein autotrophes oder methylotrophes Bakterium ist, das ausgewählt ist aus Methylophilus methylotrophus, Pseudomonas methylonica, Pseudomonas utilis, Pseudomonas inaudita, Methylomonas clara oder Alkaligenes eutropha.

**Revendications**

1. Procédé de préparation d'un matériel génétique codant pour un composé présentant par lui-même l'activité de l'interféron ou étant apte à être modifié in vivo ou in vitro pour produire un composé présentant l'activité de l'interféron, caractérisé en ce qu'il comprend au moins les étapes consistant:

(A) à effectuer la synthèse chimique de fragments du matériel génétique, chacun de ces fragments contenant quatre à six oligonucléotides inclus qui peuvent être fixés par liaison hydrogène dans l'alignement du brin complémentaire;

(B) à préparer des séquences par jonction de deux ou trois fragments appropriés qui sont pratiquement dépourvus d'intra- et d'intercomplémentarités; et

(C) à joindre lesdites séquences dans l'ordre approprié; de manière à préparer ledit matériel génétique, sous réserve que la présence de sites reconnaissables par les enzymes de restriction utilisés dans les dernières étapes du procédé soit évitée.

2. Procédé suivant la revendication 1, dans lequel les oligonucléotides utilisés dans l'étape A sont choisis entre ceux désignés ci-après par les références la à 67a:

| Désignation de l'oligo- nucléotide | Bases | Fragments | Séquences |
|---|---|---|---|
| 1a | GATCCATGTGTGAT | Aa | Ia |
| 2a | GTACACACTAGACGGCCT | | |
| 3a | CTGCCGGAGACCCAT | | |
| 4a | CTGGGTATCGGACCT | | |
| 5a | AGCCTGGATAACCGT | | |
| 6a | ATTGGCAGCATGGGA | Ba | |
| 7a | CGTACCCTGATGCTG | | |
| 8a | CTACGACGACCGTGT | | |
| 9a | CTGGCACAAATGAGC | | |
| 10a | TTACTCGGCGTAGAGA | | |
| 11a | CGCATCTCTCCGTCC | | |
| 12a | GGCAGGAGGACAGA | Ca | |
| 13a | TCCTGTCTAATGGAC | | |
| 14a | TTACCTGTCTGTACT | | |
| 15a | AGACATGACTTTGGCT | | |
| 16a | GAAACCGAAAGGGGT | | |
| 17a | TTCCCCAAGAGGAGT | | |
| 18a | TCTCCTCAAGCTACCA | Da | IIa |
| 19a | TCGATGGTAACCAGT | | |
| 20a | TTGGTCAAGGTCTTC | | |
| 21a | TCCAGAAGGCACCGG | | |
| 22a | CGTGGCCGATAGTCG | | |
| 23a | CTATCAGCGTACTCC | | |
| 24a | CATGAGGTGCTTGACT | Ea | |
| 25a | ACGAACTGATCCAAC | | |
| 26a | AGGTTGTTTAGAAG | | |
| 27a | AAATCTTCAACCTCT | | |
| 28a | TTGGAGAAATGGTGA | | |
| 29a | TTACCACTAAAGACT | | |
| 30a | TTTCTGAGTAGACGA | Fa | IIIa |
| 31a | CATCTGCTGCTTGGG | | |
| 32a | CGAACCCTGCTCCTA | | |
| 33a | ACGAGGATCTGCTAG | | |
| 34a | GACGATCTGTTTAA | | |
| 35a | ACAAATTCTGCACCGA | | |
| 36a | GACGTGGCTTGAGAT | | |
| 37a | ACTCTACCAGCAAC | | |

| Désignation de l'oligo-nucléotide | Bases | Fragments | Séquences |
|---|---|---|---|
| 38a | GGTCGTTGACTTGCT | Ga | |
| 39a | TGAACGACCTGGAAG | | |
| 40a | GGACCTTCGGACACA | | |
| 41a | CCTGTGTAATGCAAG | | |
| 42a | TTACGTTCTTCTCTC | | |
| 43a | AAGAGAGAGTGGGCG | | |
| 44a | TCACCCGCTTTGAGG | Ha | |
| 45a | AAACTCCGCTGATGAAT | | |
| 46a | CGACTACTTACGCCTGA | | |
| 47a | GCGGACTCCATCCTG | | |
| 48a | GGTAGGACCGACAAT | | |
| 49a | GCTGTTAAAAAGTAT· | | |
| 50a | TTTTCATAAAGGCAT | Ia | IVa |
| 51a | TTCCGTAGAATCACC | | |
| 52a | CTTAGTGGGACATAGA | | |
| 53a | CTGTATCTGACTGAG | | |
| 54a | CTGACTCTTTTTCATA | | |
| 55a | AAAAAGTATAGCCCG | | |
| 56a | TCGGGCACGCGAA | | |
| 57a | TGCGCTTGGGAGGTT | Ja | |
| 58a | CCCTCCAACAGGCAC | | |
| 59a | GTCCGTGCTGAGATC | | |
| 60a | GACTCTAGTACGCAA | | |
| 61a | ATGCGTTCCCTGTCTT | | |
| 62a | GGGACAGAAATAGTTGG | Ka | |
| 63a | TATCAACCAATCTTC | | |
| 64a | TTAGAAGTTCTTGCAAA | | |
| 65a | AAGAACGTTTAAGGCGC | | |
| 66a | TTCCGCGTTCCTTATTCAGCT | | |
| 67a | AAGGAATAAG | | |

3. Procédé suivant la revendication 1, dans lequel les oligonucléotides utilisés dans l'étape A sont choisis entre ceux désignés ci-après par les références 1b à 68b:

| Désignation de l'oligo-nucléotide | Bases | Fragments | Séquences |
|---|---|---|---|
| 1b | GATCCATGTGTGAT | Ab | Ib |
| 2b | GTACACACTAGACGGC | | |
| 3b | CTGCCGCAAACTCAT | | |
| 4b | GTTTGAGTATCGGACC | | |
| 5b | AGCCTGGGTAGCCGT | | |
| 6b | CATCGGCAGCGTGGGA | Bb | |
| 7b | CGCACCCTGATGCTG | | |
| 8b | CTACGACGACCGGGT | | |
| 9b | CTGGCCCAAATGCGC | | |
| 10b | TTACGCGGCATAGAG | | |
| 11b | CGTATCTCCCTGTTC | Cb | |
| 12b | GGACAAGAGGACAGA | | |
| 13b | TCCTGTCTGAAAGAC | | |
| 14b | CTTTCTGGCGGTACT | | |
| 15b | CGCCATGACTTTGGC | | |
| 16b | GAAACCGAAGGGCGT | Db | |
| 17b | TTCCCGCAGGAAGAG | | |
| 18b | CCTTCTCAAGCCATT | | |
| 19b | TTCGGTAACCAGTTC | | |
| 20b | GGTCAAGGTTTTCCG | | |

| Désignation de l'oligo-nucléotide | Bases | Fragments | Séquences |
|---|---|---|---|
| 21b | CAAAAGGCAGAAACT | Eb | IIb |
| 22b | TCTTTGATAGGGCCA | | |
| 23b | ATCCCGGTACTGCAC | | |
| 24b | TGACGTGCTTTACTA | | |
| 25b | GAAATGATTCAACAG | | |
| 26b | AGTTGTCTAAAAATT | Fb | |
| 27b | ATTTTTAACCTGTTC | | |
| 28b | GGACAAGTCGTGATT | | |
| 29b | AGCACTAAAGACTCC | | |
| 30b | TCTGAGGAGACGACG | | |
| 31b | TCTGCTGCATGGGAC | Gb | |
| 32b | TACCCTGCTTTGAGA | | |
| 33b | GAAACTCTCCTGGAC | | |
| 34b | GGACCTGTTTAAGAT | | |
| 35b | AAATTCTACACCGAAC | | |
| 36b | GTGGCTTGACATGGT | Hb | |
| 37b | TGTACCAGCAACTG | | |
| 38b | CGTTGACTTGCTGGA | | |
| 39b | AACGACCTGGAAGCC | | |
| 40b | CCTTCGGACGCAGTA | | |
| 41b | TGCGTCATCCAGGGT | Ib | IIIb |
| 42b | GGTCCCACAACCGCA | | |
| 43b | GTTGGCGTAACCGAA | | |
| 44b | TTGGCTTTGAGGCGA | | |
| 45b | ACTCCGCTGATGAAAG | | |
| 46b | CTACTTTCTTCTGAG | Jb | |
| 47b | AAGACTCCATCCTG | | |
| 48b | GTAGGACCGACAAGC | | |
| 49b | GCTGTTCGCAAATAC | | |
| 50b | GTTTATGAAGGTCG | | |
| 51b | TTCCAGCGTATCACC | Kb | |
| 52b | CATAGTGGGACATGGA | | |
| 53b | CTGTACCTGAAAGAG | | |
| 54b | CTTTCTCTTCTTTAT | | |
| 55b | AAGAAATACAGCCCG | | |
| 56b | GTCGGGCACGCGAAC | Lb | |
| 57b | TGCGCTTGGGAAGTT | | |
| 58b | CCTTCAACATGCGC | | |
| 59b | GTACGCGCTGAAATC | | |
| 60b | GACTTTAGTACGCAA | | |
| 61b | ATGCGTTCCTTCAGC | Mb | |
| 62b | GGAAGTCGGACAGGT | | |
| 63b | CTGTCCACTAACCTG | | |
| 64b | GATTGGACGTTCTTA | | |
| 65b | CAAGAATCTCTGCGT | | |
| 66b | GAGACGCATCGTTTC | | |
| 67b | AGCAAAGAATAAG | | |
| 68b | TTATTCAGCT | | |

4. Procédé suivant la revendication 1, dans lequel les fragments utilisés dans l'étape B sont choisis entre ceux désignés par les références Aa à Ka dans la revendication 2.

5. Procédé suivant la revendication 1, dans lequel les fragments utilisés dans l'étape B sont ceux choisis entre ceux désignés par les références Ab à Mb dans la revendication 3.

6. Procédé suivant la revendication 1, dans lequel les séquences utilisées dans l'étape C sont choisies entre celles désignées par les références Ia à IVa dans la revendication 2.

7. Procédé suivant la revendication 1, dans lequel les séquences utilisées dans l'étape C sont choisies entre celles désignées par les références Ib à IIIB dans la revendication 3.

8. Procédé de préparation d'une molécule d'ADN recombinant, molécule qui comprend un vecteur convenable et un gène qui code pour un composé présentant par lui-même l'activité de l'interféron ou étant apte à être modifié in vivo ou in vitro pour produire un composé présentant l'activité de l'interféon, caractérisé en ce que (i) le gène est synthétisé par un procédé suivant l'une quelconque des revendications

32

1 à 7, et (ii) le gène est lié au vecteur de sortie qu'il soit sous le contrôle transcriptionnel d'un promoteur convenable et donne par transcription une particule d'ARNm dans laquelle le codon d'initiation de . traduction de la séquence d'ARN codant pour ledit composé soit fusionné dans la région d'un site de liaison ribosomal, de sorte que la production d'un polypeptide hybride ne se produise pas.

9. Procédé suivant la revendication 6 ou 7, dans lequel le vecteur est un vecteur composite dérivé de R300B.

10. Procédé suivant la revendication 6 ou 7, dans lequel le promoteur est un promoteur *lac*.

11. Procédé suivant la revendication 6 ou 7, dans lequel le promoteur est un promoteur *trp*.

12. Procédé suivant la revendication 11, dans lequel le promoteur *trp* est un analogue synthétique d'un promoteur *trp* ΔLD102.

13. Procédé de préparation d'un micro-organisme génétiquement modifié capable d'exprimer sous forme de métabolite un composé présentant par lui-même l'activité de l'interféron ou étant apte à être modifié in vivo ou in vitro pour produire un composé présentant l'activité de l'interféron et comprenant un matériel génétique codant pour ledit métabolite, caractérisé en ce qu'il comprend les étapes consistant:

(i) à effectuer une synthèse chimique du matériel génétique par un procédé suivant les revendications 1 à 7; (ii) à synthétiser une molécule d'ADN recombinant par un procédé suivant les revendications 8 à 12; et (iii) à infecter le micro-organisme avec la molécule d'ADN recombinant.

14. Procédé suivant la revendication 13, dans lequel le micro-organisme est une bactérie autotrophe ou méthylotrophe choisie entre Methylophilus methylotrophus, Pseudomonas methylonica, Pseudomonas utilis, Pseudomonas inaudita, Methylomonas clara et Alcaligenes eutropha.

BamHI MetCys Asp Leu Pro Glu Thr His Ser Leu Asp Asn Arg Arg Thr Leu Met Leu Leu Ala Gln Met Ser

5'- GATCCATGTGTGATCTGCCCGAGACCCATAGCCTGGATAACCGTCGTACCCTGATGCTGCTGGCACAAATGAGC - 3'
3'- GTACACACTAGACGGGCCTCTGGGTATCGGACCTATTGGCAGCATGGGACTACGACGACCGTGT - 5'

Arg Ile Ser Pro Ser Ser Cys Leu Met Asp Arg His Asp Phe Gly Phe Pro Gln Glu Glu Phe Asp Gly Asn Gln

5'- CGCATCTCTCCGTCCTCCTGTCTAATGGACAGACATGACTTTGGCTTTCCCCAAGAGGAGTTCGATGGTAACCACT - 3'
3'- TTACTCGGCGTAGAGAGGCAGGAGGACAGATTACCTGTCTGTACTGAAACCGAAAGGGGTTCTCCTCAAGCTACCA - 5'

Phe Gln Lys Ala Pro Ala Ile Ser Val Leu His Glu Leu Ile Gln Gln Ile Phe Asn Leu Phe Thr Thr Lys Asp

5'- TCCAGAAGGCACCGGCTATCAGCGTACTCCACGAACTGATCCAACAAATCTTCAACCTCTTTACCACTAAAGACT - 3'
3'- TTGGTCAAGGTCTTCCGTGGCCGATAGTCGCATGAGGTGCTTGACTAGGTTGTTTAGAAGTTGGAGAAATGGTGA - 5'

Ser Ser Ala Ala Trp Asp Glu Asp Leu Leu Asp Lys Phe Cys Thr Glu Leu Tyr Gln Gln Leu Asn Asp Leu Glu

5'- CATCTGCTGCTTGGGACGAGGATCTGCTAGACAAATTCTGCACCGA[A]CTCTACCAGCAACTGAACGACCTGGAAG - 3'
3'- TTTCTGAGTAGACGACGAACCCTGCTCCTAGACGATCTGTTTAAGACGTGGCT[T]GAGATGGTCGTTGACTTGCT - 5'

Ala Cys Val Met Gln Glu Glu Arg Val Gly Glu Thr Pro Leu Met Asn Ala Asp Ser Ile Leu Ala Val Lys Lys Tyr

5'- CCTGTGTAATGCAAGAAGAGAGAGTGGGCGAAACTCCGCTGATGAATGCGGACTCCATCCTGGCTGTTAAAAAGTAT - 3'
3'- GGACCTTCGGACACATTACGTTCTTCTCTCTCACCCGCTTTGAGGCGACTACTTACGCCTGAGGTAGGACCGACAAT - 5'

Phe Arg Arg Ile Thr Leu Tyr Leu Thr Glu Lys Lys Tyr Ser Pro Cys Ala Trp Glu Val Val Arg Ala Glu Ile

5'- TTCCGTAGAATCACCCTGTATCTGACTGAGAAAAAGTATAGCCCGTGCGCTTGGGAGGTTGTCCGTGCTGAGATC - 3'
3'- TTTTCATAAAGGCATCTTAGTGGGACATAGACTGACTCTTTTTCATATCGGGCACGCGAACCCTCCAACAGGCAC - 5'

Met Arg Ser Leu Ser Leu Ser Thr Asn Leu Gln Glu Arg Leu Arg Arg Lys Glu Stop Sal I

5'- ATGCGTTCCCTGTCTTTATCAACCAATCTTCAAGAACGTTTAAGGCGCAAGGAATAAG - 3'
3'- GACTCTAGTACGCAAGGGACAGAAATAGTTGGTTAGAAGTTCTTGCAAATTCCGCGTTCCTTATTCAGCT - 5'

# FIG.1

MetCys Asp Leu Pro Gln Thr His Ser Leu Gly Ser Arg Arg Thr Leu Met Leu Leu Ala Gln Met Arg

5'- GATCCAT GT GT GAT CT GCCGCAAACT CAT AGCCT GGGT AGCCGT CGCACCCT GAT GCT GCT GGCCCAAAT GCGC -3'

3'- GT ACACACT AGACGGCGTTT GAGT AT CGGACCCAT CGCCAGCGT GGGACT ACGACGACCGCGTTT ACGCGGCAT AGAG -5'

Arg Ile Ser Leu Phe Ser Cys Leu Lys Asp Arg His Asp Phe Gly Phe Pro Gln Glu Glu Phe Gly Asn Gln Phe

5'- CGT AT CT CCCT GTT CT CCT GT CT GAAAGACCGCCAT GACTTT GGCTT CCCGCAGCAAGAGT T CGGT AACCAGTT C -3'

3'- GGACAAGAGGACAGACTTT CT GGCGGT ACT GAAACCGAAGGGCGT CCTT CT CAAGCCATT GGT CAAGGTTTT CCG -5'

Gln Lys Ala Glu Thr Ile Pro Val Leu His Glu Met Ile Gln Gln Ile Phe Asn Leu Phe Ser Thr Lys Asp Ser

5'- CAAAAGGCAGAAACT AT CCCGGT ACT GCACGAAAT GATT CAACAGATTTTT AACCT GTT CAGCACT AAAGACT CC -3'

3'- TCTTT GAT AGGGCCAT GACGT GCTTT ACT AAGTT GT CT AAAAATT GGACAAGT CGT GATTT CT GAGGAGACGACG -5'

Ser Ala Ala Trp Asp Glu Thr Leu Leu Asp Lys Phe Tyr Thr Glu Leu Tyr Gln Gln Leu Asn Asp Leu Glu Ala

5'- TCT GCT GCAT GGGACGAAACT CT CCT GGACAAATT CT ACACCGAACT GT ACCAGCAACT GAACGACCT GGAAGCC -3'

3'- TACCCT GCTTT GAGAGGACCT GTTT AAGAT GT GGCTT GACAT GGT CGTT GACTT GCT GGACCTT CGGACGCAGT A -5'

Cys Val Ile Gln Gly Val Gly Val Thr Glu Thr Pro Leu Met Lys Glu Asp Ser Ile Leu Ala Val Arg Lys Tyr

5'- T GCGT CAT CCAGGGT GTT GGCGT AACCGAAACT CC GCT GAT GAAAGAAGACT CCAT CCT CGCT GTT CGCAAAT AC -3'

3'- GGT CCCACAACCGCATT GGCTTT GAGGCGACT ACTTT CTT CT GAGGT AGGACCGACAACCGTTT AT GAAGGT CC -5'

Phe Gln Arg Ile Thr Leu Tyr Leu Lys Glu Lys Lys Tyr Ser Pro Cys Ala Trp Glu Val Val Arg Ala Glu Ile

5'- TT CCAGCGT AT CACCCT GT ACCT GAAAGAGAAGAAAT ACAGCCCGT GCGCTT GGGAAGTT GT ACGCGCCT GAAAT C -3'

3'- CAT AGT GGGACAT GGACTTT CT CTT CTTT AT GT CGGGCACGCGAACCCTT CAACAT GCGCGACTT AGT ACGCAA -5'

Met Arg Ser Phe Ser Leu Ser Thr Asn Leu Gln Glu Ser Leu Arg Ser Lys Glu Stop Sal I

5'- ATGCGTT CCTT CAGCCT GT CCACT AACCT GCAAGAAT CT CT GCGT AGCAAAGAAT AAG -3'

3'- GGAAGT CGGACAGGT GATT GGACGTT CTT AGAGACGCAT CGTTT CTT ATT CAGCT -5'

## FIG. 2

TRANSCRIPTION
CONTROL

promoter

operator

3'DNA
5'

5' AUG 3'mRNA

ribosome
binding
site

coding
sequence

initiation
codon

TRANSLATION
CONTROL

FIG. 3

BamHI cleavage

- G3'
- CCTAG5'

5'GATCC —
3'G —

"fill-in" by
DNA PolymeraseI

- GGATC3'
- CCTAG5'

5'GATCC —
3'CTAGG —

Ligation to AluI
Lac promoter
fragment

- GGATCCT
- CCTAGGA

BamHI

lac promoter

AGGATCC —
TCCCTAG —

BamHl

FIG. 4

Transplantation
Fragment

LacOP

E

B

Synthetic
IFgene

Ap^r

S

pPM50
(3.4kb)

Rep

FIG. 5

3

EP 0 062 971 B1

EcoRI 5'- AATTCT GGCAAAT ATTCT GAAAT GAGCT GTT GACAAT TAAT CAT CGAACT AGTT AACT AGT ACGCAAGTT CACGT AAAAAGGGT AT -3' Taq I
3'- GACCGTTTAT AAGACTTTACTCGACAACTGTTAATTAGTAGCTTGATCAATTGATCATGCGTTCAAGTGCATTTTTCCCATAGC-5'

## FIG. 6

Vector = EcoRI/ClaI cut pAT153

Ap^r

Tc^r

EcoRI

GAATTC
CTTAAG

ClaI/TaqI

ATCGAT
TAGCTA

Synthetic trp Promoter

## FIG. 7

FIG.8

FIG.IO

FIG.II

FIG.9

Monomer Anion

Pyridine/triethylamine

H⁺

Dimer Anion

+ Monomer anion

Trimer

Trimer anion

B = Protected base

DMTr = 4,4'-dimethoxytrityl

FIG.12

EP 0 062 971 B1

FIG.13

FIG.14

| | | | | | | |
|---|---|---|---|---|---|---|
| O | 200 | 400 | 600 | 800 | 1000 | 1200 | pcIF 24.40 |

pcIF 24.47

PstI    PvuII    PvuII    EcoRI    PvuII    PstI

| O | 200 | 400 | 600 | 800 | 1000 |
|---|---|---|---|---|---|

pcIF 29.43

PstI PvuII    PvuII    EcoRI    PvuII PstI

| O | 200 | 400 |
|---|---|---|

pcIF 41.13

PstI    BglII PvuII    BglII    PstI

| 200 | 400 | 600 |
|---|---|---|

pcIF 56.2

PstI    AluI    PstI

FIG.15

```
                 met leu thr val leu pro val leu ser
G31T CTA GTC ACT ATG CTC ACT GTT CTC CCC GTC TTG TCC

phe his asp ileu asn gln cys pro ala val ileu thr leu
TTC CAT GAC ATT AAT CAA TGC CCG GCT GTC ATT ACT CTT

gly arg cys ala his asp ala met thr val ser
GGC CGG TGT GCT CAT GAT GCT ATG ACT GTA AGT GT.......
```

FIG.16